# EUROPEAN PATENT APPLICATION

(11) **EP 2 735 874 A1**
(43) Date of publication of application: **28.05.2014**
(21) Application number: 12382458.3
(22) Date of filing: 21.11.2012
(51) Int. Cl.: G01N 33/574

(54) **Methods of diagnosing and therapeutic agents for use in the treatment of prostate cancer**

(71) Applicant: Fundación Para La Investigación Biomédica Del Hospital Universitario Puerta De Hierro, 28222 Majadahonda - Madrid (ES)
(72) Inventor: Álvarez Vallina, Luis, 28200 San Lorenzo de el Escorial - Madrid (ES); Sánchez Martín, David, 37900 Santa Marta de Tormes - Salamanca (ES)
(74) Representative: ABG Patentes, S.L.

(57) **Abstract**

The present invention relates to the use of PA28, which is over-expressed in prostate cancer tissue, as a marker suitable for diagnostic test for prostate cancer, and to therapeutic agents specific for PA28 for use in the treatment of prostate cancer.

SEQUENCE LISTING

<110>  Fundación para la Investigación Biomédica del Hospital
       Universitario Puerta de Hierro Majadahonda

<120>  METHODS OF DIAGNOSING AND THERAPEUTIC AGENTS FOR USE IN THE
       TREATMENT OF PROSTATE CANCER

<130>  P9008EP00

<160>  39

<170>  PatentIn version 3.5

<210>  1
<211>  7
<212>  PRT
<213>  Artificial Sequence

<220>
<223>  010H01 antibody CDR1

<400>  1

Gly Asp Asn Leu Ile Asp Thr
1               5


<210>  2
<211>  8
<212>  PRT
<213>  Artificial Sequence

<220>
<223>  010H01 antibody CDR2

<400>  2

Ile Glu Asp Asn Asp Gly Ser Thr
1               5


<210>  3
<211>  22
<212>  PRT
<213>  Artificial Sequence

<220>
<223>  010H01 antibody CDR3

<400>  3

Ala Gly Asp Trp Tyr Ser Tyr Phe Ala Val Arg Phe Ala Asp Val Arg
1               5                   10                  15


Asp Thr Lys Val Lys Ser
            20


<210>  4
<211>  7
<212>  PRT
<213>  Artificial Sequence

<220>
<223>  011A10 antibody CDR1

<400>  4

Gly Phe Ser Val Asn Asp Phe
1               5


<210>  5
<211>  8
<212>  PRT
<213>  Artificial Sequence

<220>
<223>  011A10 antibody CDR2

<400>  5

Ile Ala Ala Asn Asn Gly Ser Thr
1               5


<210>  6
<211>  15
<212>  PRT
<213>  Artificial Sequence

<220>
<223>  011A10 antibody CDR3

<400>  6

Ala Gly Glu Gly Trp Ser Ala Ala Gly Glu Asp Thr Met Arg Ser
1               5                   10                  15


<210>  7
<211>  8
<212>  PRT
<213>  Artificial Sequence

<220>
<223>  010D03 antibody CDR1

<400>  7

Gly Asp Arg Val Ser Tyr Asp Asp
1               5


<210>  8
<211>  8
<212>  PRT
<213>  Artificial Sequence

<220>
<223>  010D03 antibody CDR2

<400>  8

Ile Glu Asn Pro Asp Gly Ser Thr
1               5

<210>  9
<211>  21
<212>  PRT
<213>  Artificial Sequence

<220>
<223>  010D03 antibody CDR3

<400>  9

Ala Gly Asn Thr Ser Asp Leu Trp Asp His Gly Trp Ala Lys Arg Thr
1               5                   10                  15

Lys Glu Val Pro Phe
            20

<210>  10
<211>  7
<212>  PRT
<213>  Artificial Sequence

<220>
<223>  011D12 antibody CDR1

<400>  10

Gly Phe Thr Phe Thr Ala Ala
1               5

<210>  11
<211>  8
<212>  PRT
<213>  Artificial Sequence

<220>
<223>  011D12 antibody CDR2

<400>  11

Ile Arg Asn Gln Asn Gly Ser Thr
1               5

<210>  12
<211>  11
<212>  PRT
<213>  Artificial Sequence

<220>
<223>  011D12 antibody CDR3

<400>  12

Ala Ala Ala Phe Gly Ala Ser Pro Ile Gly Ser
1               5                   10

<210>  13
<211>  8
<212>  PRT
<213>  Artificial Sequence

<220>
<223>  011H12 antibody CDR1

<400>  13

Gly Tyr Arg Val Ile Thr Glu Phe
1               5


<210>  14
<211>  8
<212>  PRT
<213>  Artificial Sequence

<220>
<223>  011H12 antibody CDR2

<400>  14

Ile Asn Thr Gln Asn Gly Ser Thr
1               5


<210>  15
<211>  12
<212>  PRT
<213>  Artificial Sequence

<220>
<223>  011H12 antibody CDR3

<400>  15

Ala Ser Ser Val Glu Glu Lys Asn Ala Leu Arg Tyr
1               5                   10


<210>  16
<211>  7
<212>  PRT
<213>  Artificial Sequence

<220>
<223>  i11D12 antibody CDR1

<400>  16

Gly Asp Thr Phe Ile His Asp
1               5


<210>  17
<211>  8
<212>  PRT
<213>  Artificial Sequence

<220>
<223>  i11D12 antibody CDR2

<400>  17

Ile Arg Glu Gln Asn Gly Ser Thr
1               5

<210>  18
<211>  13
<212>  PRT
<213>  Artificial Sequence

<220>
<223>  i11D12 antibody CDR3

<400>  18

Ala Thr Ser Val Gly Pro Ala Pro Tyr His Met Tyr Phe
1               5                   10


<210>  19
<211>  7
<212>  PRT
<213>  Artificial Sequence

<220>
<223>  020D11 antibody CDR1

<400>  19

Gly Phe Met Leu Asn Tyr Tyr
1               5


<210>  20
<211>  8
<212>  PRT
<213>  Artificial Sequence

<220>
<223>  020D11 antibody CDR2

<400>  20

Ile Arg Gly Asp Ser Gly Ser Thr
1               5


<210>  21
<211>  13
<212>  PRT
<213>  Artificial Sequence

<220>
<223>  020D11 antibody CDR3

<400>  21

Ala Thr Thr Ser Trp Ser Gly Glu His His Phe Glu Tyr
1               5                   10


<210>  22
<211>  7
<212>  PRT
<213>  Artificial Sequence

<220>
<223>  010G05 antibody CDR1

<400>  22

Gly Tyr Lys Val Arg Pro Asp
1               5


<210>  23
<211>  8
<212>  PRT
<213>  Artificial Sequence

<220>
<223>  010G05 antibody CDR2

<400>  23

Ile Glu Gly Gln Gly Gly Ser Thr
1               5


<210>  24
<211>  12
<212>  PRT
<213>  Artificial Sequence

<220>
<223>  010G05 antibody CDR3

<400>  24

Ala Arg Phe Ser Leu Cys Gln Asp Ala Met Gly Tyr
1               5                   10


<210>  25
<211>  8
<212>  PRT
<213>  Artificial Sequence

<220>
<223>  010D06 antibody CDR1

<400>  25

Gly Tyr Asn Phe Ser Asp Glu Asp
1               5


<210>  26
<211>  8
<212>  PRT
<213>  Artificial Sequence

<220>
<223>  010D06 antibody CDR2

<400>  26

Ile Cys Gly Ala Asn Gly Ser Thr
1               5


<210>  27
<211>  15
<212>  PRT
<213>  Artificial Sequence

<220>
<223>  010D06 antibody CDR3

<400>  27

Ala Ala Ser Lys Asp Cys Met Leu Glu Glu Tyr Ser Met Lys Ser
1               5                   10                  15


<210>  28
<211>  7
<212>  PRT
<213>  Artificial Sequence

<220>
<223>  010A05 antibody CDR1

<400>  28

Arg Arg Ala Tyr Arg Ser Asp
1               5


<210>  29
<211>  8
<212>  PRT
<213>  Artificial Sequence

<220>
<223>  010A05 antibody CDR2

<400>  29

Ile Arg Thr Ser Asn Gly Ser Thr
1               5


<210>  30
<211>  12
<212>  PRT
<213>  Artificial Sequence

<220>
<223>  010A05 antibody CDR3

<400>  30

Ala Gly Val Ala Thr Val Thr Ser Glu Leu Thr Tyr
1               5                   10


<210>  31
<211>  8
<212>  PRT
<213>  Artificial Sequence

<220>
<223>  020F08 antibody CDR1

<400>  31

Tyr Ala Lys Leu Ser Asp Gln Asn
1               5

<210>  32
<211>  8
<212>  PRT
<213>  Artificial Sequence

<220>
<223>  020F08 antibody CDR2

<400>  32

Ile Arg Asn Ser Asn Gly Ser Thr
1               5


<210>  33
<211>  15
<212>  PRT
<213>  Artificial Sequence

<220>
<223>  020F08 antibody CDR3

<400>  33

Ala Arg Leu Leu Val Pro Thr Leu Val Ala Thr Thr Leu Thr Phe
1               5                   10                  15


<210>  34
<211>  8
<212>  PRT
<213>  Artificial Sequence

<220>
<223>  BGal antibody CDR1

<400>  34

Gly Val Ser Ile Thr Ala Glu Ser
1               5


<210>  35
<211>  8
<212>  PRT
<213>  Artificial Sequence

<220>
<223>  BGal antibody CDR2

<400>  35

Ile Thr Met Arg Asp Gly Ser Thr
1               5


<210>  36
<211>  16
<212>  PRT
<213>  Artificial Sequence

<220>
<223>  BGal antibody CDR3

<400>  36

Ala Arg Ala Arg Ala Met Tyr Pro Leu Arg Ser Ser Gln Leu Glu Ser
1               5                   10                  15


<210>  37
<211>  30
<212>  DNA
<213>  Artificial Sequence

<220>
<223>  ClaI-H12  primer

<400>  37
tcatcgatgg aggtgcagct ggtggagtct                                        30


<210>  38
<211>  29
<212>  DNA
<213>  Artificial Sequence

<220>
<223>  ClaI-CEA1  primer

<400>  38
caacgcgtca tggctcaggt gcagctggt                                         29


<210>  39
<211>  29
<212>  DNA
<213>  Artificial Sequence

<220>
<223>  NotI-CEA1 primer

<400>  39
tggtgggcgg ccgctgagga gacggtgac                                         29

## Description

### FIELD OF THE INVENTION

The invention falls within the field of diagnosis and, more specifically, in the field of diagnosis of prostate cancer by means of using a marker protein the expression of which is over-expressed in prostate tissue samples and biological fluids. The invention also relates to therapeutic agents for use in the treatment of a subject suffering from prostate cancer.

### BACKGROUND OF THE INVENTION

Prostate cancer (prostate cancer) is the most common type of cancer in the Western male population, where it is responsible for more male deaths than any other cancer, except lung cancer. Even though the introduction of the prostate specific antigen (PSA) test in the late 80s of the past century has led to a dramatic increase its detection (Jemal et al., 2007, Cancer statistics, 57:43-66), the risk of developing this type of cancer during a lifetime is estimated at one in six men, and the risk of death due to metastatic prostate cancer is 1 in 30.

The diagnostic tools for detecting prostate cancer can be separated into those that screen for the disease, such as PSA and digital rectal exams (DRE), and the decisive diagnosis set of transrectal ultrasound guided prostate biopsies (TRUS).

One of the limitations of serum PSA as a tumour marker is its lack of specificity, which results in a high rate of negative biopsies. There is a substantial overlap in the serum PSA values of men with benign prostatic hyperplasia (BPH) and those with prostate cancer. Elevated PSA levels can also be attributed to other factors such as prostatitis, prostate irritation, and recent ejaculation (Pannek & Partin, 1997, Oncology 11:1273-8; Loeb, 2009, Urol. Oncol. 27:64-6).

Various attempts have been made to overcome the limitations of PSA screening, including the use of Ratio PSA, age-adjusted PSA cut-off points, free-PSA, PSA-density, PSA-velocity, PSA-slope and PSA-doubling time, and (-2) proPSA (Mikolajczyk et al., 2004, Clin Biochem. 37:519-28; Sokoll et al., 2008, J. Urol 180:539-43; Morote et al., 1997, J. Urol. 158:502-4). All have been proposed as a means of improving serum PSA specificity in the detection of prostate cancer. Nevertheless, there has been no evidence to suggest that any of these testing strategies improves health outcomes (Harris & Lohr; 2002, Ann. Intern. Med. 137:917-29).

Furthermore, the expression of PSMA is not restricted to prostate cancer, and its expression has been shown on tumour-associated neovasculature in several types of malignant neoplasms, tumour cells and only in prostate adenocarcinoma (Chang et al. 1999, Cancer Research 59:3192-98). As a consequence of the current limitations of PSA and PSMA-based screening tests, there is a need to find markers that show higher specificity for prostate cancer. A number of studies have explored the possibilities of using different markers in a number of tumour types, some of which are proteins associated with the proteasome.

Proteasomes are large protein complexes that are located in the nucleus and cytoplasm of eukaryotic cells. The function of proteasomes is to degrade unneeded or damaged proteins by proteolysis. Proteasomes are part of an important mechanism by which cells regulate the concentration of certain proteins and degrade misfolded proteins those. Structurally, the proteasome is a cylindrical complex containing a "core" of four stacked rings around a central pore. Proteasome subcomponents are often referred to by its sedimentation coefficient Svedberg (designated S). The most common form of the proteasome is known as the 26S proteasome, which contains a core particle structure 20S and 19S two regulatory subunits. An alternative way is the regulatory subunit called 11S subunit, which can be associated with the core essentially in the same way that the 19S particle. The 11S subunit may play a role in the degradation of short peptides and foreign objects, such as those produced after infection by a virus. This structure is also referred as PA28 or REG.

WO 2002/082076 describes tumour markers that can be used for screening, diagnosis and identification of subtypes of renal cell carcinoma. Among the markers described is PA28α(PSME1).

WO 2006/108094 describes the use of tumour markers for homing for the diagnosis of liver metastases of colorectal cancer (CRC) in liver and the prognosis of patients with colorectal cancer. Among the tumour markers of directing to liver is PA28α (PSME1).

WO 2003/068054 discloses methods for the classification of ovarian tumours in BRCA1, BRCA2 or non-BRCA types, by means of the expression levels of different ovarian tumour markers, including PA28β or PSME2. Longuespée et al. (2012, Histochem Cell Biol 138:141-54) describe the C-terminal fragment of the 11S proteasome activator complex (PA28α, Reg alpha) as a specific marker of early and late stages of ovarian cancer. The results revealed that the immunohistochemical localization of nuclear and cytoplasmic fragment was in ovarian cancer samples, and nuclear benign ovarian tumours.

Wang et al. (2009, BMC Genomics 10:383) have revealed that PA28 is up-regulated in processes of carcinogenesis by comparing several cell lines of oral squamous cell carcinoma (Oral Squamous Cell Carcinoma, OSCC) and oral keratinocytes. Zhang et al. (2011, J Proteome Res 10:2863-72) have also identified a series of proteins that were over-expressed in esophageal squamous cell carcinoma in cell lysates of tissue specimens, including PA28α (PSME1).

WO 2003/063773 discloses that the gene encoding PA28β (PSME2), among other molecules, is under-regulated in prostate cancer.

Therefore, there is an urgent need to find alternative tumour markers that make possible the detection of prostate cancer.

### BRIEF DESCRIPTION OF THE FIGURES

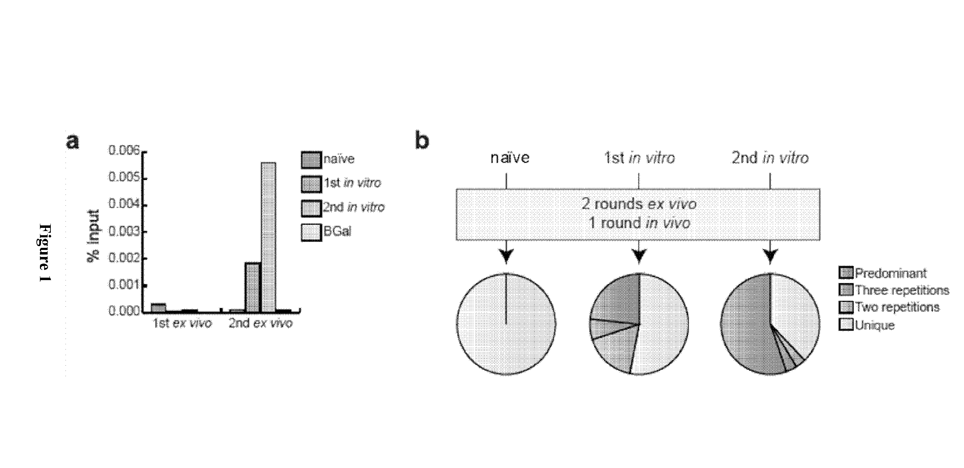

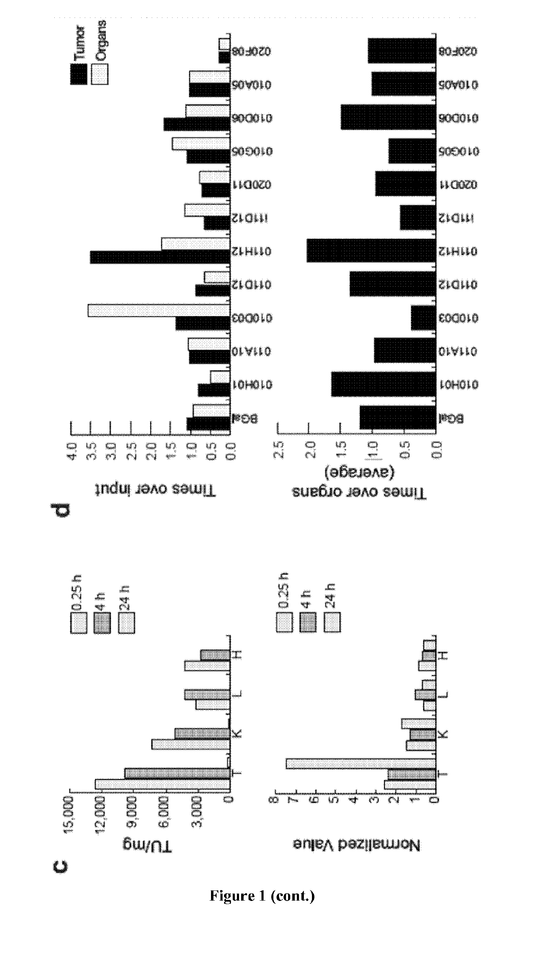

**Figure 1****. Enrichment and diversity loss.** The recovery of phage increases as the *ex vivo* selections progress. The recovery is greater if the repertoire was previously enriched *in vitro* (a). However, the use of previously enriched repertoires greatly reduces the available diversity (b). (c) Influence of the circulation time: Using as input an *ex vivo* enriched repertoire, the total recovery of infective particles is maximum at 15 min, and drops gradually until 24 h (left panel). This is so for all the organs analyzed. However, as the time of circulation increases there is a higher specific recovery from the tumour (right panel; T, Tumour, K, kidney, L, liver, H, heart). (d) Competition experiments using mixtures of fresh monoclonal phage: after 24 h of circulation, the phage were recovered from the organs or the tumour, and the percentage of each was compared with that present in the input (upper panel) or with the average of the organs (lower panel).

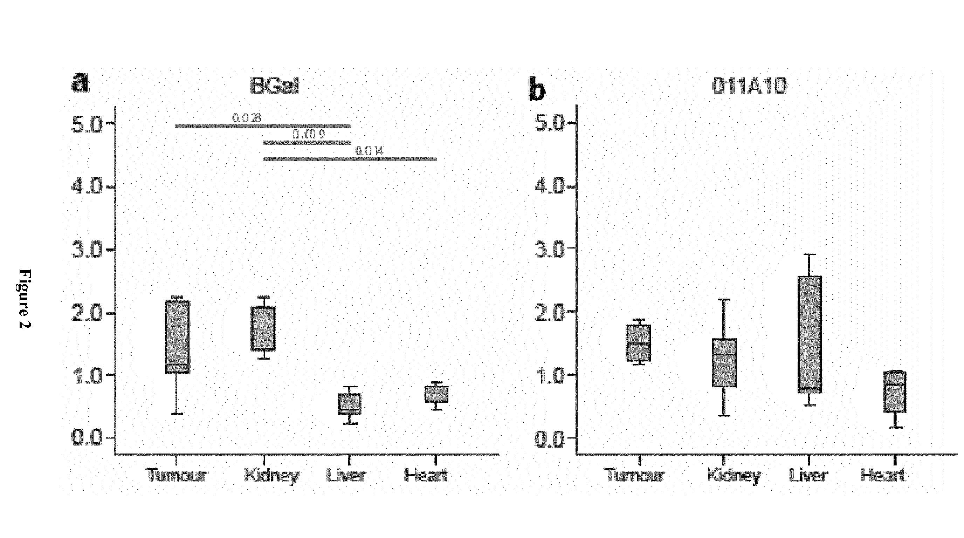

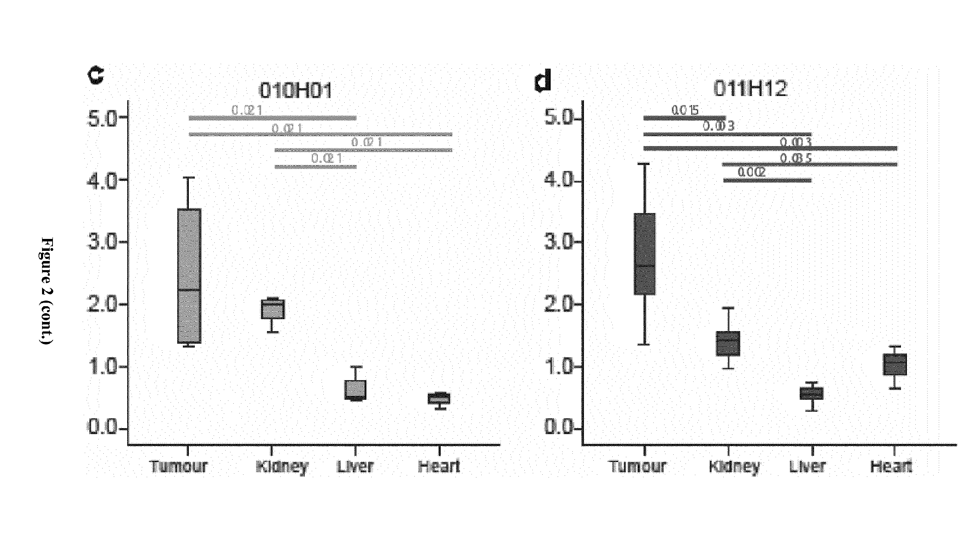

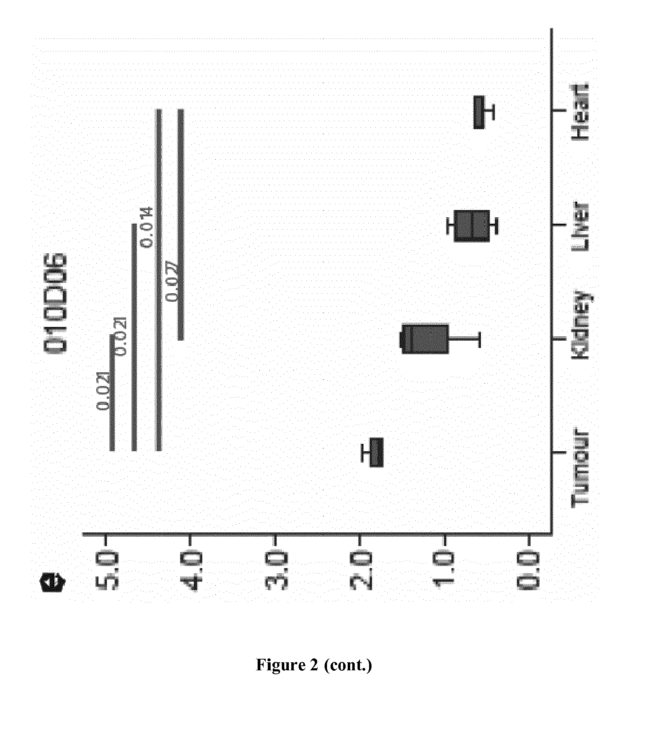

**Figure 2****. Homing of monoclonal phage.** Normalized values showing the differential accumulation of different phage clones (N = 5 mice per group). (a) BGal phage used as a reference: the expected retention for a phage whose target is not present in the tissue. Different clones that show a specific retention in the tumour (c-e), and a specific retention in all the organs (b). Significant differences (p-value) U Mann-Whitney.

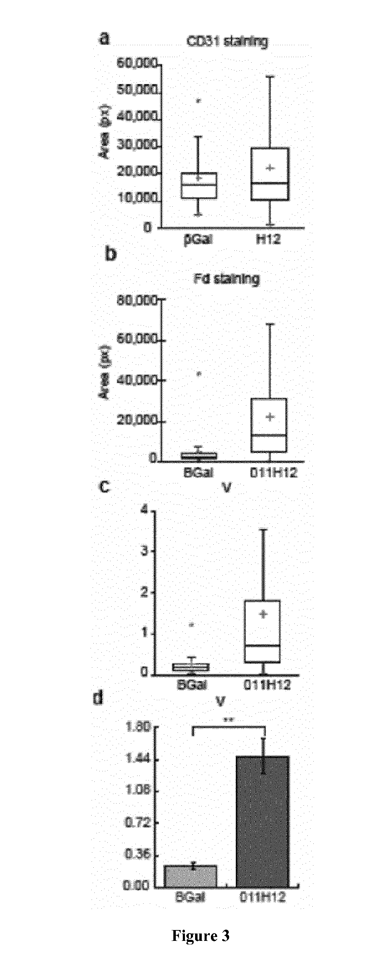

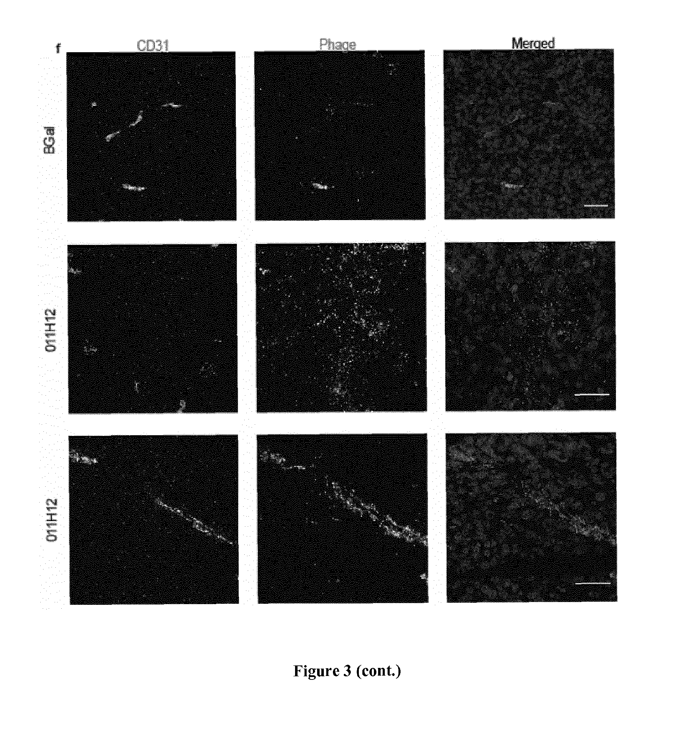

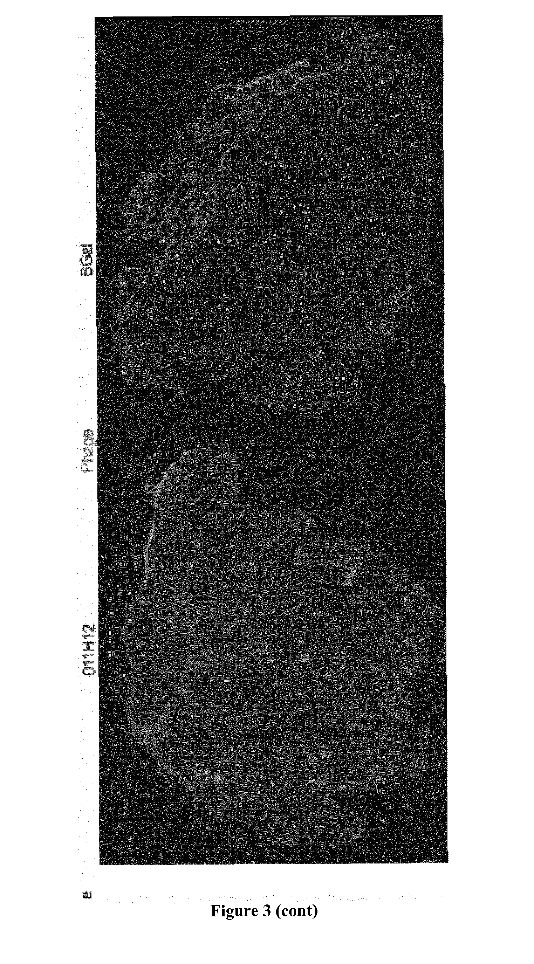

**Figure 3****. Tumour-specific homing of 011H12 phage.** Sections from tumours that have received the phage (either the reference BGal or 011H12) were analyzed by immunofluorescence and the areas positive for CD31 (red) and the phage (green) were quantified. No difference was found in the total area positive for endothelial markers (a). There was a higher phage staining in the 011H12 clone (b). The V parameter [(c) total phage/total endothelium] was 6 times higher in the 011H12 clone (d). **Significant differences (p < 0.0001) U Mann-Whitney. (e) Heterogeneous distribution of the phage. The 011H12 phage accumulates specifically in the tumour, as compared to the reference phage. In both cases, there is an unspecific retention of the phage in the rim of the tumour. (left) The distribution of the 011H12 phage is heterogeneous in the section: there are main deposits that spread from a vessel, and minor deposits in wide stromal areas. (right). (f) The distribution of the reference phage (BGal) is restricted to the inner side of some vessels, as well as the edge of the tumour. The reference phage (Bgal, top row) is trapped inside CD31-positive vessel structures; whereas the 011H12 phage is localized not only inside the vessels, but also in wide areas of stroma (middle row) and in the outer area of endothelial vessels (low row). Scale bar is 50 µm.

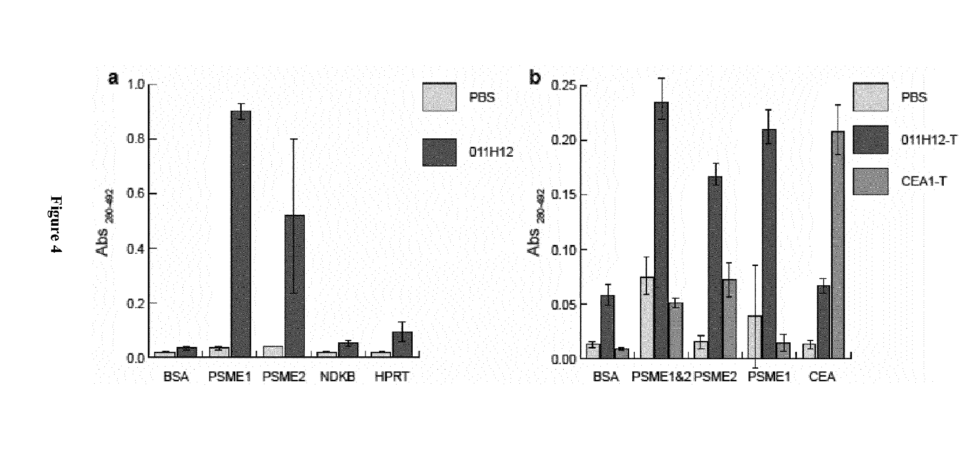

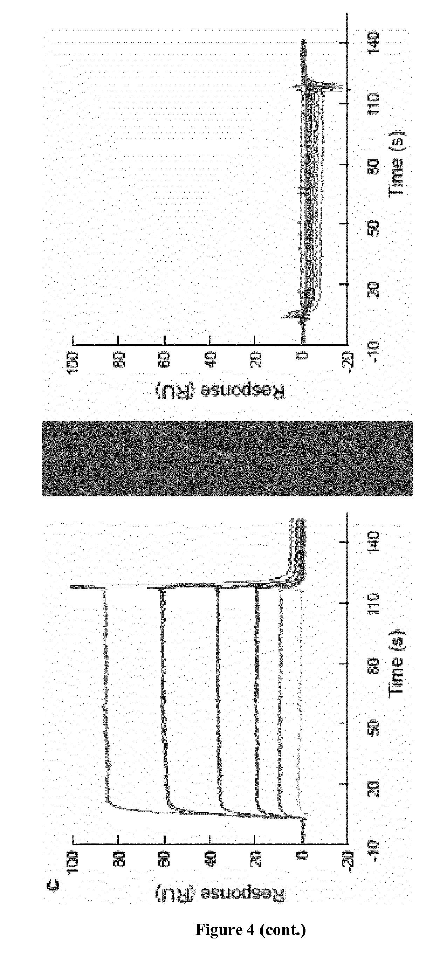

**Figure 4****. Specific interaction of 011H12 antibody with PA28 subunits.** Purified soluble domain antibodies (a) were tested against a panel of candidates. 011H12 interacts with PSME1 and slightly less with PSME2. This interaction may be dependent on a complex epitope present in the multimeric PA28 as the signal increases when combining PSME1 and PSME2 and a trimeric format (b) of the antibody (mean ± SD). SPR analysis of antibody binding to PSME1 immobilized on the sensor chip surface (c). Overlay of sensorgrams obtained by the injection of the soluble purified antibodies 011H12 (left) or CEA1 (right) at different concentrations (lower to upper curve: 62.5, 125, 250, 500, 1000 and 2000 nM). Identical concentrations are displayed with the same colors. Each measurement was performed in duplicate. An empty flow cell and a HRPT coated surface were used as reference surfaces. The corresponding background responses were subtracted.

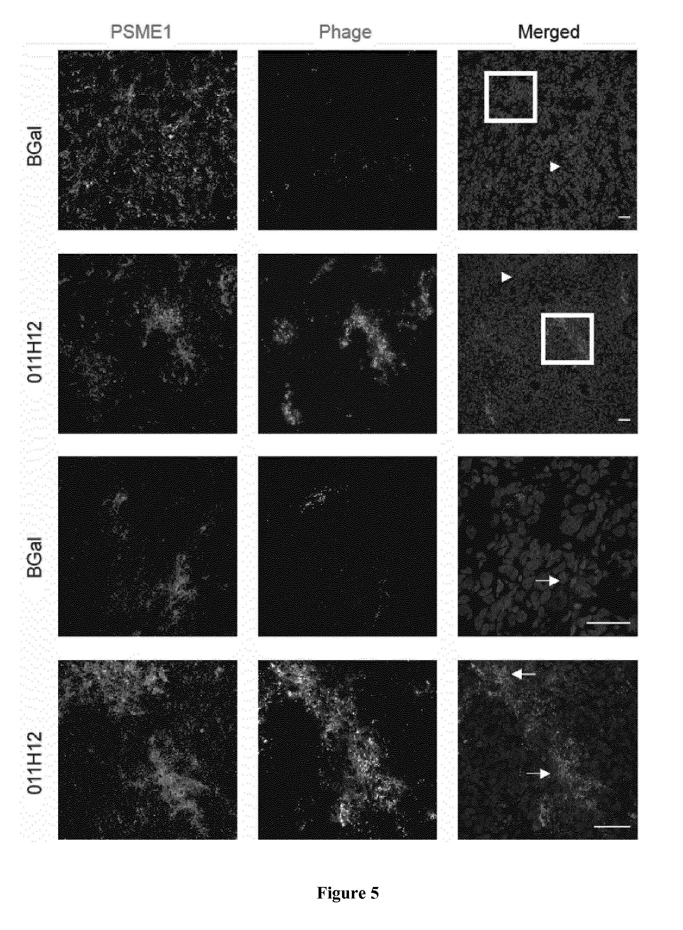

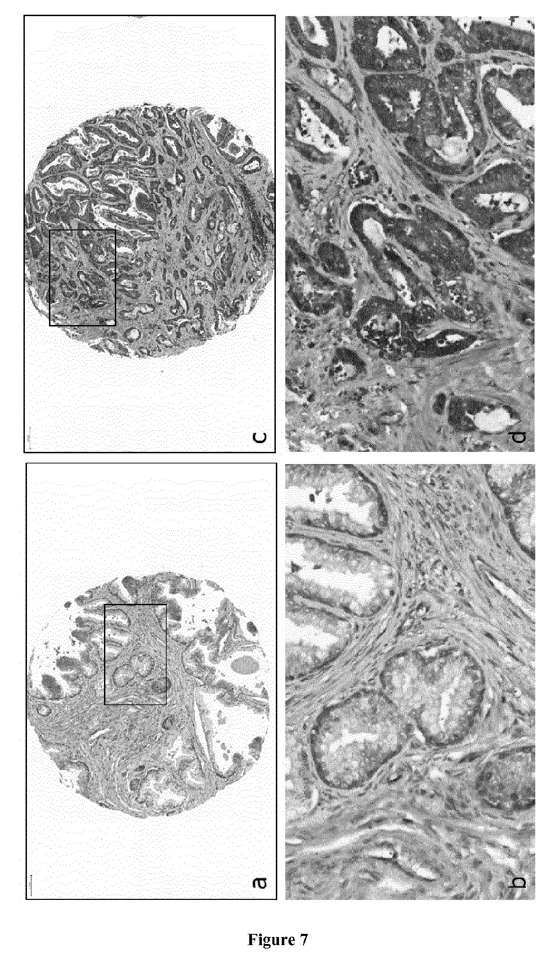

**Figure 5****. Co-localization of *in vivo* administered 011H12 phage with PSME1 in PPC-1 tumour xenografts.** PSME1 is heterogeneously distributed: most of the blood vessels seem to be positive, and there are wide areas that seem to be unrelated to CD31 structures (Supplementary Fig. 7). The reference phage (BGal, 1st and 3rd rows) colocalizes inside PSME1-positive vessel structures; whereas the 011H12 phage (2nd and 4th rows) is localized not only inside these PSME1-positive vessels (white arrowheads), but also in wide areas of stroma where there is no co-localization of the reference phage and PSME1 (white arrows). Scale bar is 50 µm (red: PSME1, green: phage).

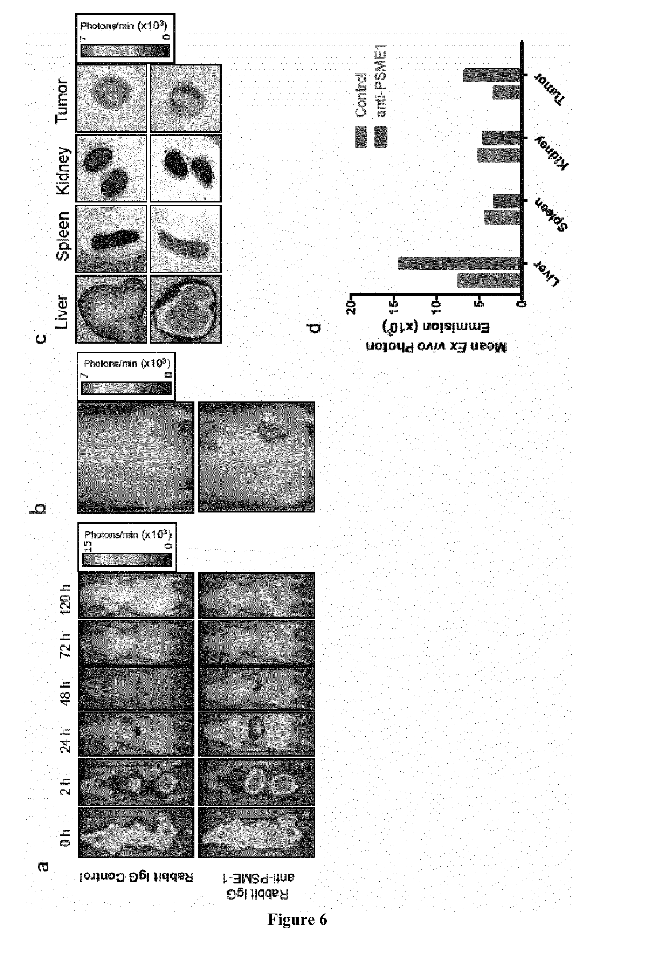

**Figure 6****. Tumour homing of anti-PSME1 antibodies.** Near-infrared fluorescence imaging of nude mice bearing PPC-1 tumour xenografts. (A) Ventral view shows similar accumulation of both rabbit polyclonal anti-PSME1 antibody and control rabbit IgG in the bladder. (B) Control IgG did not localize in the tumour, whereas anti-PSME1 antibody showed a specific signal. *Ex vivo* imaging (C) and quantification (D) of the organs (liver, kidney, spleen and tumour).
**Figure 7****. Immunohistochemical detection of PSME1 in human prostate sections using tissue microarrays.** (A) low-magnification (x15) image of a TMA spot with normal prostatic glands showing strong staining in basal epithelial cells. (B) higher magnification view (x63) of representative region from A showing the staining in basal epithelial cells, vascular endothelium and in surrounding stromal areas. (C) low-magnification (x15) image of a TMA spot with prostate cancer showing a widespread and more intense PSME1 staining. (D) higher magnification view (x63) of representative region from C showing strong staining in carcinoma glands (basal and luminal epithelial cells), vascular endothelial cells and stroma. Scale bars: (A, C) 100 µm; (B, D) 20 µm.

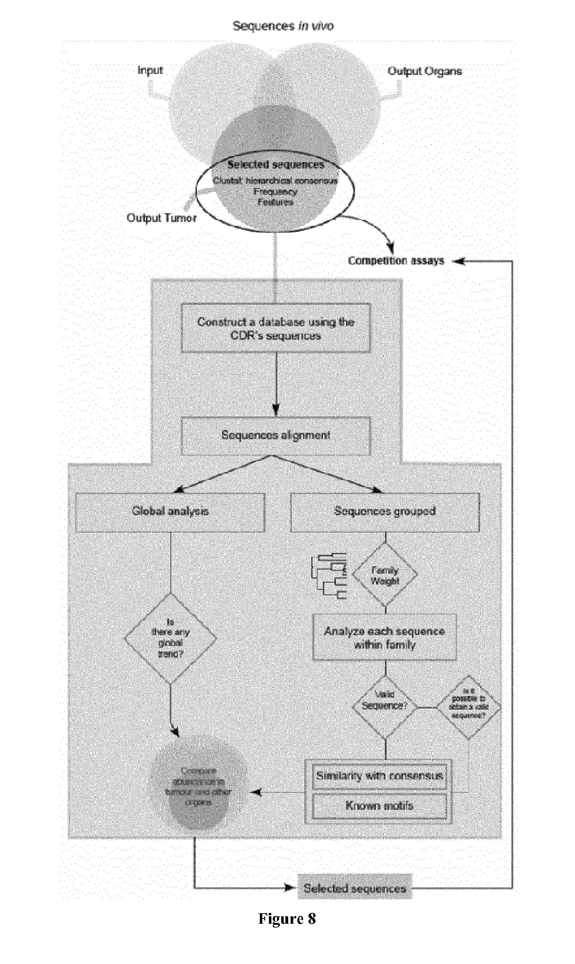

**Figure 8****. Decision tree.** A database was constructed containing the information of the CDR's and organ for each sequence retrieved. Sequences were aligned using ClustalW2 (Larkin et al., 2007, Bioinformatics 23:2947-8) and grouped and visualized using Jalview (Waterhouse et al., 2009, Bioinformatics 25:1189-91). The weight of each family was calculated as the percentage of the sequences in it. Each sequence within a family was tested for a valid sequence (following the parameters of number (if present) of amber stop codons and the first nucleotide after each; as well as calculated protein parameters (number of cysteines, instability index, etc.), if the sequence was not valid, an alternative valid sequence was searched inside the family [the information was discarded if no alternative sequence could be found]). All valid sequences were selected according to the comparison with the consensus sequence inside their family and with known motifs present in the literature (RGD, CendR, NGR, etc.). The frequency of preselected families and sequences was compared in the input, output in reference organs and output in tumour. Selected individual sequences were further analyzed in competition assays.

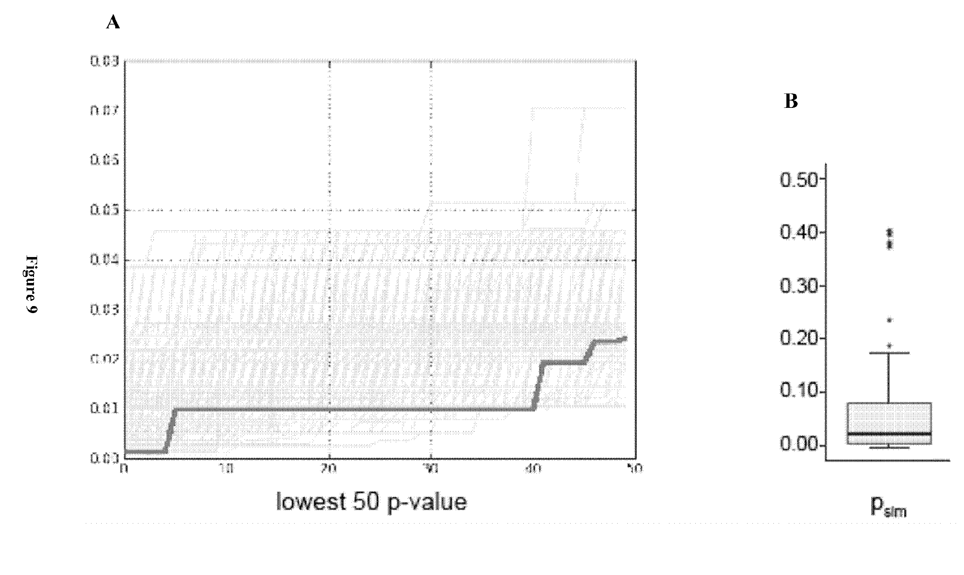

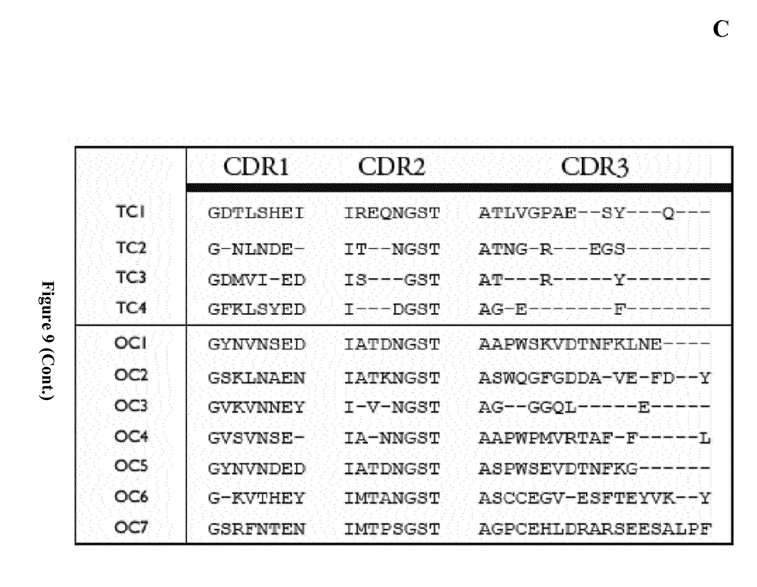

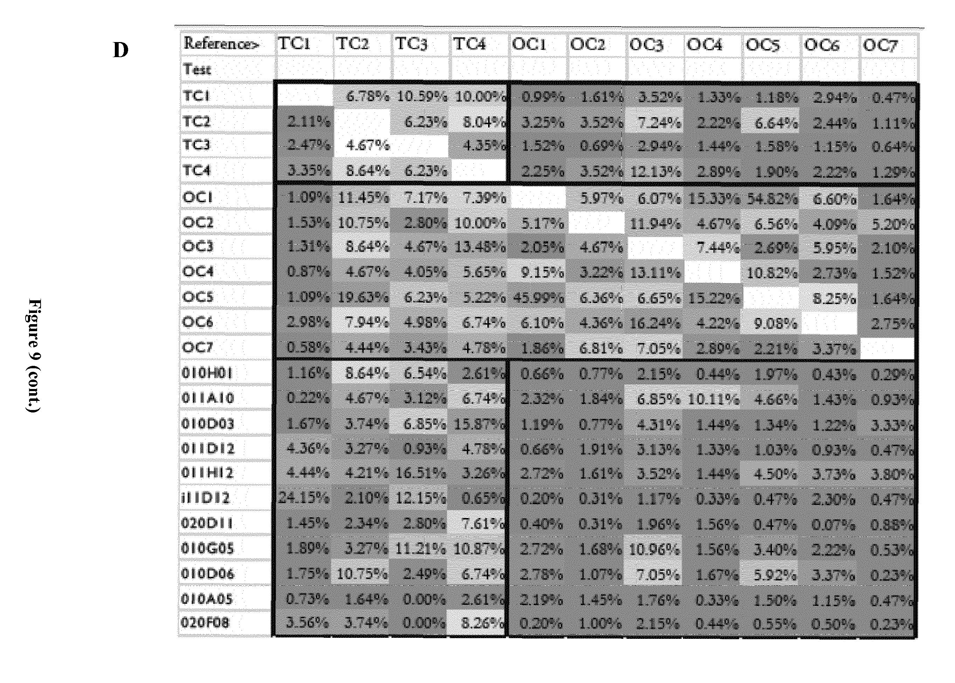

**Figure 9****. Computational analysis of the sequences.** (A) Resampling-based evaluation assessing selectivity (tumour vs. combined organs) of the combination of three residues and their corresponding position within the CDR's after an *in vivo* selection of a pre-enriched repertoire (round 0.2.0). The plot of the experimental smallest p-values (red line) determined by Fisher's exact test was compared with the corresponding simulated datasets (light gray). Plotted are the 50 smallest p-values (index number of p-values 1 through 50, ascending order) generated in each of the 1000 permutations. (B) For each of the 50 smallest p-values, a new p-value (pₛᵢₘ) was calculated. Each pₛᵢₘ represents the probability of the experimental p-values to lay within the simulated distribution, and they were calculated as the proportion of simulated p-values smaller than the experimental p-values. The 50 pₛᵢₘ were plotted in a box plot, with median 0.024. (C) A similar analysis was performed for the combination of all *in vivo* rounds of selection. The combination of residues and their respective position that were significantly (p-value < 0.05) different between the tumour and the combined organs let us calculate different consensus sequences with preferential accumulation in the tumour (TC) and consensus sequences absent from the tumour (OC). (D) Heatmap based on the evaluation of the selected sequences (Table 1) *a posteriori* for common three residues with the consensus sequences.

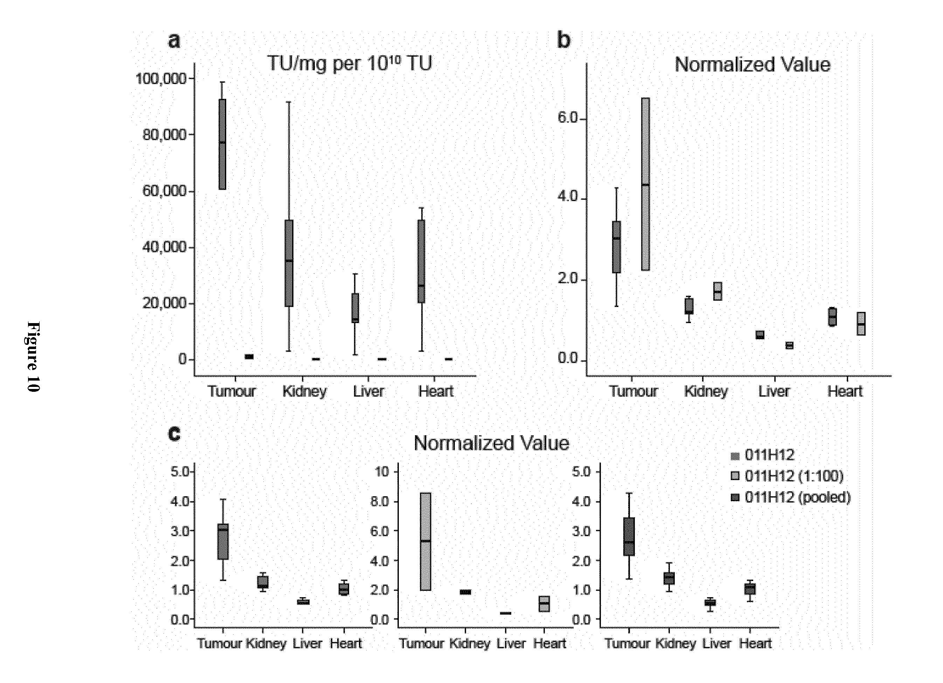

**Figure 10****. Usefulness of the normalized value.** Using as input either 1 × 10¹¹ TU of monoclonal phage (*n* = 5) or 1 × 10⁹ TU (*n* = 2), the total recovery of infective particles is highest for the highest dose, from all organs (A). However, the normalized value follows approximately the same pattern (B), and can be even pooled into a new variable for the same monoclonal phage (C).

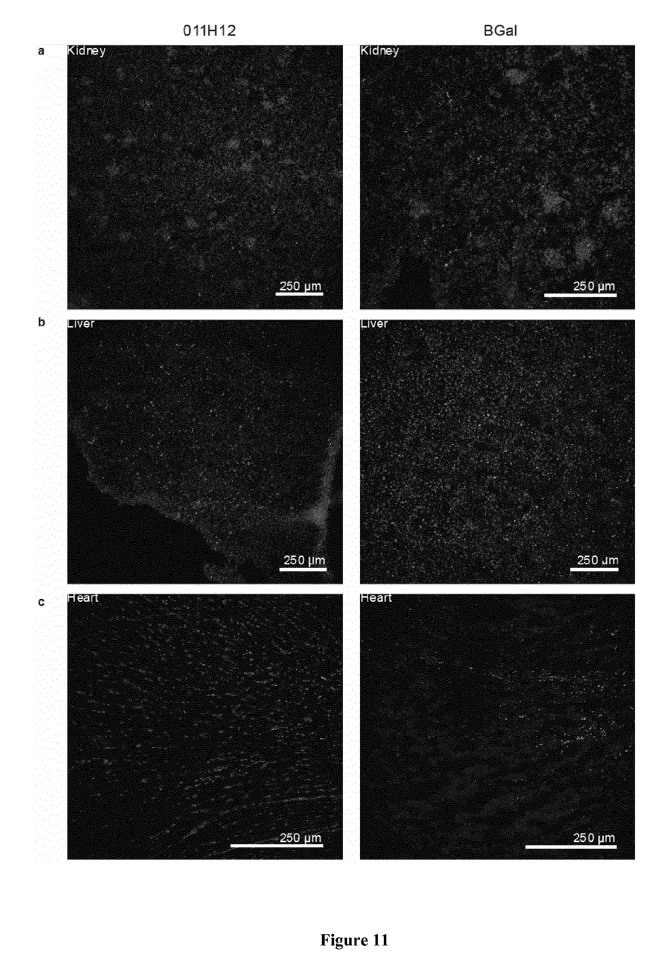

**Figure 11****. Immunofluorescence in non-tumour organs.** The reference phage (BGal) and the 011H12 phage shared a similar localization in non-tumour organs, mostly confined in vessel structures in the kidney (A) and heart (C), or scattered throughout the liver (B) (red: CD31, green: phage).

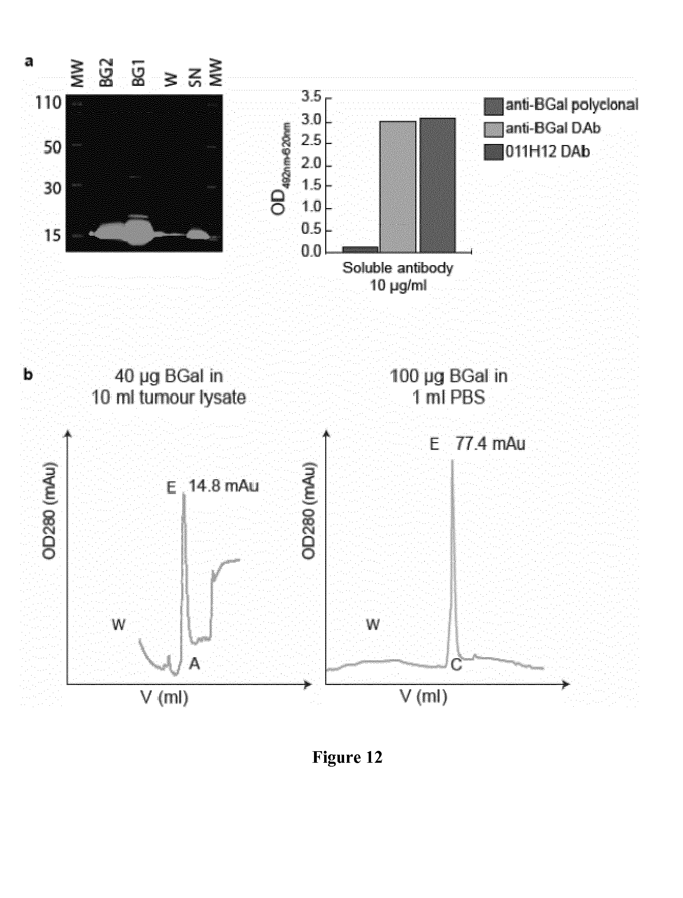

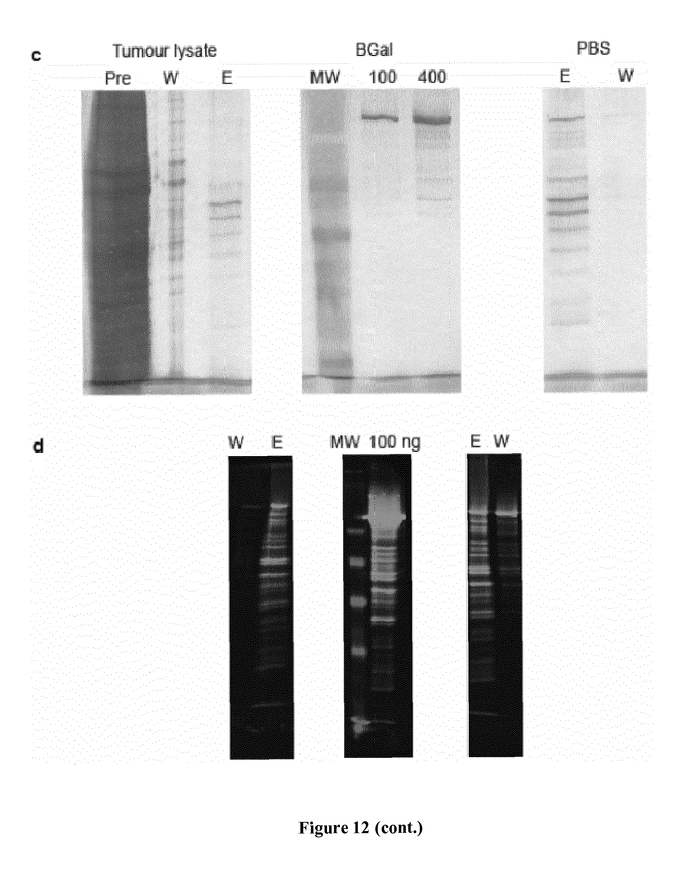

**Figure 12****. Purification of the antigen using immobilized antibodies.** The anti-β-galactosidase DAb was purified from *E. coli* supernatant using protein A (a, left panel; MW: Molecular weight, BG1, BG2: eluted fractions, W: washing fraction, SN: raw supernatant) and tested against the immobilized antigen (b-galactosidase) in ELISA (a, right panel). Different quantities of commercial b-galactosidase were diluted in PBS (100 mg in 1 ml PBS) or in tumour lysate (or 40 mg in tumour lysate) and purified using a column with immobilized anti-β-galactosidase DAb as a proof of concept. Approximately 60% of the sample diluted in the tumour lysate (b, left panel) and 80% of the sample diluted in PBS (b, right panel) were recovered. The recovered fractions were analyzed by silver staining (c; Pre: sample, W: washing fraction, E: eluted fraction, MW: molecular weight, 100: 100 ng of commercial b-galactosidase, 400: 400 ng of commercial β-galactosidase) and were tested in western blotting with an anti-β-galactosidase polyclonal antibody (d). It is possible to purify the antigen using this strategy, even if it is degraded (c, d).

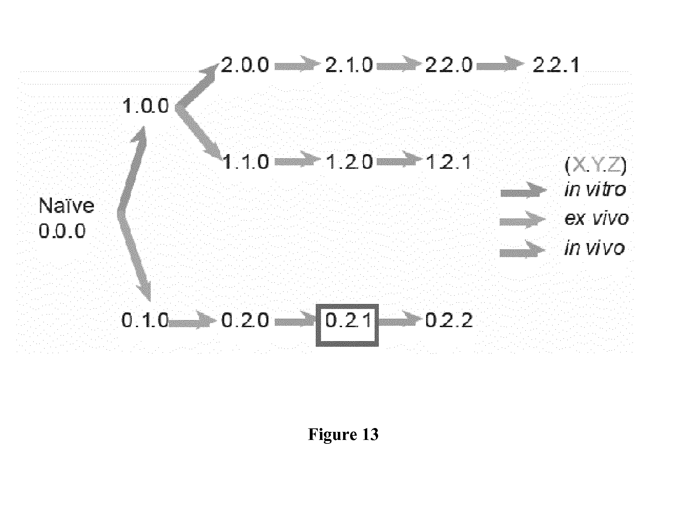

**Figure 13****. Summary of the rounds of selection explored.** The flow of the selections starts in the naive repertoire and follows *an in vitro* (blue arrow), *ex vivo* (green arrow) or *in vivo* (orange arrow) selection path. The three digits code represents the number of *in vitro* (X, first number), *ex vivo* (Y, second number) or *in vivo* (Z, third number) selections. Sample clones were sequenced for each round of selection (-3000 clones sequenced: 95 from the input, 95 from the target output, and 96 from reference organs - *in vivo* selections). We have explored several combinations of rounds of selection, but focused on the one outlined in the paper (red box) because of the enrichment and the diversity available.

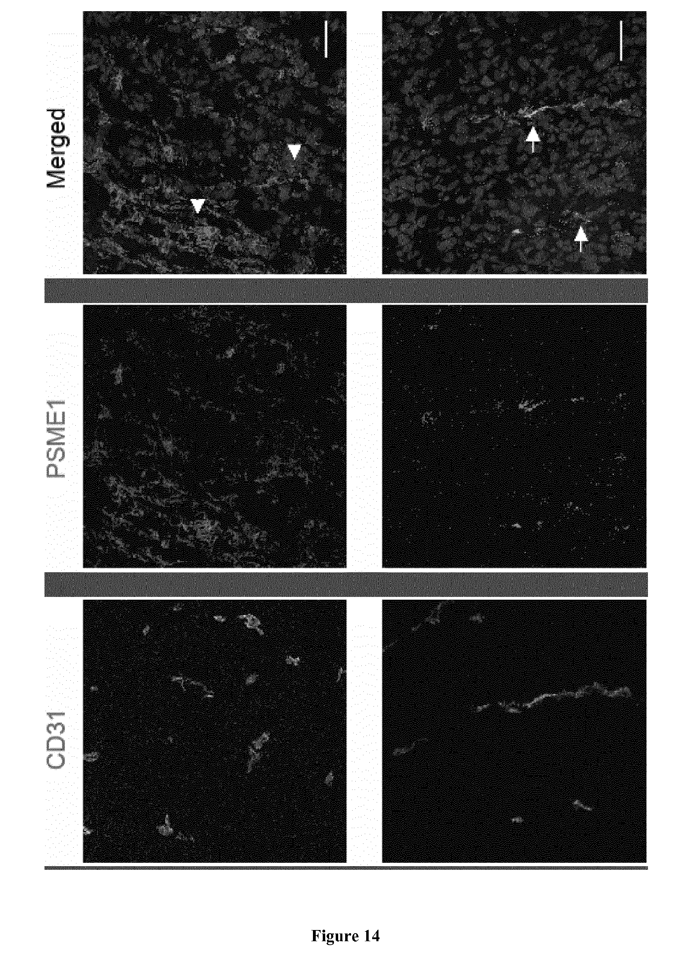

**Figure 14****. Distribution of PSME1 and CD31 in PPC-1 tumour xenografts.** There are two distribution patterns of PSME-1 (red: CD31, green: PSME-1): either associated to endothelium (white arrows) or in clusters in the stroma (white arrowheads). Scale bar is 50 µm.

### DETAILED DESCRIPTION OF THE INVENTION

The authors of the present invention have isolated antibodies that specifically recognize PA28, particularly the PA28α or PSME1 subunit and the PA28β or PSME2 subunit on the surface of prostate cancer cells. These antibodies were obtained by a method of phage display selection with a combination of *in vivo* and *ex vivo* selections to determine this new cellular location for PA28. They have also showed that PSME1 is overexpressed in tissue specimens from patients with prostate cancer.

The present invention therefore relates to the use of PA28 as a tumour marker of prostate cancer. The invention also relates to methods for diagnosing prostate cancer in a subject and to therapeutic agents for use in the treatment of prostate cancer.

### Definitions

The meaning of some terms and expressions as they are used in the present description are indicated below to aid in understanding.

The term "PA28" or "REG", as used herein, refers to the proteasome activator 28 protein. PA28 is a ring-shaped multimeric complex (180 kDa) that can bind to the two ends of the 20S proteasome and dramatically stimulate its capacity to hydrolyze small peptides. In mammals, PA28 is a heteromeric complex of two homologous subunits, PA28α (PSME1) and PA28β (PSME2), both of which are induced by γ-interferon, which is a potent stimulator of MHC-class I antigen presentation. Most peptides presented on surface MHC-molecules are generated by proteasomes during the course of intracellular protein degradation. PA28 is assumed to promote antigen presentation

The term "PSME1" or "PA28α", as used herein, refers to the proteasome activator PA28 alpha subunit also known as proteasome activator complex subunit 1, 11S regulator complex subunit alpha, REG-alpha, activator of multicatalytic protease subunit 1, interferon gamma up-regulated I-5111 protein, and IGUP I-5111. The human isoforms of PSME 1 are depicted under accession numbers CAG33061.1 and EAW66106.1 in the GenBank database.

The term "PSME2" or "PA28β", as used herein, refers to the proteasome activator PA28 beta subunit also known as proteasome activator complex subunit 2, 11S regulator complex subunit beta, REG-beta, and activator of multicatalytic protease subunit 2. The human isoforms of PSME 2 are depicted under accession numbers CAG46543.1 and EAW66101.1 in the GenBank database.

### Methods for diagnosing prostate cancer

The authors of the present invention have determined that PA28, which is a ubiquous protein normally located in the cytoplasm and nucleus of eukaryotic cells, is also found on the cell surface on prostate cancer cells. This is an unexpected finding that facilitates the diagnosis of prostate cancer in a subject.

Thus, in a first aspect, the present invention relates to *an in vitro* method for diagnosing prostate cancer in a subject, hereinafter "first diagnostic method of the invention", comprising detecting the presence of at least one subunit of PA28 in a prostate tissue sample from said subject, wherein the detection of said at least one subunit of PA28 on the cell surface is indicative of said subject having prostate cancer.

The expression "method for diagnosing", as used herein, relates to a method that may essentially consist of the previously mentioned steps or may include additional steps. However, it must be understood that the method is carried out *in vitro,* i.e., it is not carried out in the human or animal body. "Diagnosing", as used herein, relates to evaluating the probability according to which a subject suffers from a disease. As it will be understood by persons skilled in the art, such evaluation, although it is preferred that it is, normally may not be correct for 100% of the subjects to be diagnosed. The term, however, requires that a statistically significant part of the subjects can be identified as suffering from the disease or having a predisposition for it. The person skilled in the art can determine if a part is statistically significant without further delay by using several well known statistic evaluation tools, for example, determination of confidence intervals, determination of the p value, Student's t-test, Mann-Whitney test, etc. The details are in Dowdy and Wearden, Statistics for Research, John Wiley & Sons, New York 1983. The preferred confidence intervals are of at least 50%, at least 60%, at least 70%, at least 80%, at least 90%, at least 95%, preferably of at least 99%. The p values are preferably 0.2, 0.1, 0.05, and preferably 0.01. The method for diagnosing of the invention allows assessing whether a subject is afflicted with prostate cancer.

The term "prostate cancer", as used herein, refers to any cancer of the prostate gland in which cells of the prostate mutate and begin to multiply out of control irrespective of the stage of the cancer. The extent to which prostate cancer has progressed in a patient is assessed taking into account clinical and histopathological information. The stage of cancer is classified based on the TNM system, taking into account tumour size (T), whether there is lymph node involvement (N), the presence of metastasis (M), and the tumour grading (G). A tumour classed as T1 is confined to the prostate gland and too small to be felt by digital rectal examination. T1 further includes T1a (fewer than 5% cancerous cells in tissue sample) and T1b (more than 5%) subdivisions. T1c indicates the patient has an elevated Prostate Specific Antigen (PSA; see definition later). If the tumour is large enough to be felt during a digital rectal examination, it is classified as T2. T2a means only one side of the prostate gland (left or right) is involved; T2b means both sides have a tumour(s). T2 is commonly termed "localized cancer". If the cancer is T3, it has spread to the connected tissue near the prostate (T3a) or the seminal vesicles (T3b). T4 indicates cancer spread to tissue next to the prostate, for example the bladder sphincter, rectum or pelvis wall. The prostate cancer may also spread into the regional lymph nodes of the pelvis and this is assessed as N1 stage of prostate cancer. These stages of T3, T4 and N1 are collectively termed "locally advanced" or regional cancer. If the cancer has spread to distant sites, such as the bone, it is said to be "metastasized" or at the M1 stage. Prostate cancer that has spread to distant lymph nodes is categorized as M1 a while that which has spread to bone is M1b and that which has spread to organs such as liver or brain is assessed as M1c. If left untreated, prostate cancer almost universally metastasizes to bone, although it can also spread to liver and lungs.

Like staging, grading levels are also assigned to prostate cancer cases. Grading takes place after a biopsy (removal and examination of tissue) is done. The grade refers to the cancer's appearance and indicates the rate of growing. Most pathologists grade prostate cancer according to the Gleason score, which assigns a grade from 1 to 5 based on how the cancerous cells look compared to normal prostate cells:
- Grade 1: cancerous tissue looks very much like normal prostate cells.
- Grades 2 to 4: some cells look like normal prostate cells, while others do not. Patterns of cells in these grades vary.
- Grade 5: cells do not look like normal prostate cells and they appear to be scattered haphazardly throughout the prostate.

The higher the Gleason score, the more likely it is that the cancer will grow and spread rapidly. Pathologists often identify the two most common patterns of cells in the tissue, assign a Gleason grade to each, and add the two grades. The result is a number between two and 10. A Gleason score of less than six indicates a less aggressive cancer. A grade seven and up is considered more aggressive.

The term "subject", as used herein, refers to any animal susceptible of suffering from prostate cancer. In a particular embodiment, the subject is a mammal and includes, but is not limited to, domestic and farm animals, primates and humans, for example, human beings, non-human primates, cows, horses, pigs, sheep, goats, dogs, cats, or rodents. In a preferred embodiment, the subject is a male human being of any age or race.

In a first step, the first diagnostic method of the invention comprises detecting the presence of at least one subunit of PA28 in a prostate tissue sample from said subject, wherein the detection of said at least one subunit of PA28 on the cell surface is indicative of said subject having prostate cancer.

The methods for detecting the presence of protein are well-known by a person skilled in the art and include a number of alternatives. Virtually any conventional method which allows detecting or determining the presence of at least one subunit of PA28 can be used in putting the first method of the invention into practice. By way of a non-limiting example, said detection can be carried out using antibodies with the capacity for binding specifically to the protein to be detected (or to fragments thereof containing the antigenic determinants) and subsequent detection of the resulting antigen-antibody complexes.

The term "antibody" is used herein in the sense of its capacity to bind specifically to an antigen and thus, it refers to a molecule having such capacity: Included within said term are:
- an intact antibody that binds specifically to the target antigen;
- an antibody fragment that binds specifically to the target antigen; and
- an antibody-like molecule that binds specifically to the target antigen.

As used herein, the term "intact antibody" refers to an immunoglobulin molecule capable of specific binding to its cognate target, including a target, such as a carbohydrate, polynucleotide, lipid, polypeptide, etc., through at least one binding recognition site (e.g., antigen binding site), including a site located in the variable region of the immunoglobulin molecule. An antibody includes an antibody of any class, namely IgA, IgD, IgE, IgG (or sub-classes thereof), and IgM, and the antibody need not be of any particular class. In a preferred embodiment, the antibody is an IgG.

As used herein, the term "antibody fragment" refers to functional fragments of antibodies, such as Fab, Fab', F(ab')₂, Fv, single chain (ScFv), heavy chain or fragment thereof, light chain or fragment thereof, a domain antibody (DAb) (i.e., the variable domain of an antibody heavy chain (V_{H} domain) or the variable domain of the antibody light chain (V_{L} domain)) or dimers thereof, V_{H} or dimers thereof, V_{L} or dimers thereof, nanobodies (camelid V_{H}), and functional variants thereof, fusion proteins comprising an antibody, or any other modified configuration of the immunoglobulin molecule that comprises an antigen recognition site of a desired specificity. An antibody fragment may refer to an antigen binding fragment. In a preferred embodiment, the antibody fragment is a V_{H} or domain antibody or DAb.

Techniques for the preparation and use of the various antibodies are well known in the art (Ausubel et al., ed., Current Protocols in Molecular Biology, John Wiley and Sons, Inc., NY 1987-2001; Sambrook, et al., Molecular Cloning: A Laboratory Manual, 2nd Edition, Cold Spring Harbor, NY, 1989; Harlow and Lane, Antibodies, a Laboratory Manual, Cold Spring Harbor, NY, 1989; Colligan, et al., ed., Current Protocols in Immunology, John Wiley and Sons, Inc., NY 1994-2001; Colligan et al., Current Protocols in Protein Science, John Wiley and Sons, NY, NY, 1997-2001; Kohler et al., Nature 256:495-497, 1975; US 4, 816, 567, Queen et al., Proc. Natl. Acad. Sci. 86:10029-10033, 1989). For example, fully human monoclonal antibodies lacking any non-human sequences can be prepared from human immunoglobulin transgenic mice or from phage display libraries.

Also included within the definition of "antibody" according to the instant invention is an antibody-like molecule that binds specifically to the target antigen. As used herein, the term "antibody-hke molecule" or "non-immunoglobulin alternative binding_protein" refers to binding proteins other than immunoglobulin, that are based on different protein topologies or scaffolds. The term scaffold is meant to describe a protein framework that can carry altered amino acids or sequence insertions that confer on protein variants different functions, usually for binding specific targets. Examples of such non-immunoglobulin alternative binding proteins or scaffolds are well known in the art, and include without limitation DARPins, monobodies, anticalins, affibodies, avimers, and protein scaffolds reviewed in Binz et al., 2005 (Nat. Biotech. 23:1257-68), included herein by reference.

As used herein, the term "functional variant of an antibody" refers to a protein resulting from the insertion, deletion or substitution of one or more amino acid residues from the sequence of said antibody, and that substantially maintains its capacity to bind to its cognate target, and wherein said antibody is selected from the group consisting of an intact antibody, an antibody fragment and an antibody-like molecule. Functional variants of an antibody according to the invention include polypeptides showing a sequence identity with the sequence of said antibody of at least 50%, at least 60%, at least 70%, at least 80%, at least 90%, at least 91%, at least 92%, at least 93%, at least 94%, at least 95%, at least 96%, at least 97%, at least 98%, or at least 99%. Likewise, functional variants of an antibody according to the invention will preferably have a capacity to bind to its cognate target of at least 50%, at least 60%, at least 70%, at least 80%, at least 90%, at least 91%, at least 92%, at least 93%, at least 94%, at least 95%, at least 96%, at least 97%, at least 98%, or at least 99% of the capacity to bind to the cognate target of said antibody.

As used herein, the term "functional fragment of an antibody" also refers to a protein with identical sequence as that of said antibody, wherein said antibody is selected from the group consisting of an intact antibody, an antibody fragment and an antibody-like molecule, with a deletion of at least 1 amino acid, at least 2 amino acids, at least 3 amino acids, at least 4 amino acids, at least 5 amino acids, at least 10 amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids, at least 100 amino acids, at least 200 amino acids, at least 500 amino acids, or at least 1000 amino acids from its N-terminus, and/or a deletion of at least 1 amino acid, at least 2 amino acids, at least 3 amino acids, at least 4 amino acids, at least 5 amino acids, at least 10 amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids, at least 100 amino acids, at least 200 amino acids, at least 500 amino acids, or at least 1000 amino acids from its C-terminus. The deletions will preferably be at the C-terminus of the antibody. Functional fragments of an antibody will preferably have a capacity to bind to its cognate target of at least 50%, at least 60%, at least 70%, at least 80%, at least 90%, at least 91%, at least 92%, at least 93%, at least 94%, at least 95%, at least 96%, at least 97%, at least 98%, or at least 99% of the capacity to bind to its cognate target.

For use in the instant invention, the "antibody" is an antibody which specifically binds to at least one subunit of PA28. Thus, in a particular embodiment, the antibody is selected from the group consisting of
- an intact antibody that binds specifically to at least one subunit of PA28;
- an antibody fragment that binds specifically to at least one subunit of PA28; and
- an antibody-like molecule that binds specifically to at least one subunit of PA28.

The antibodies suitable for detecting the presence of at least one subunit of PA28 can be, for example, polyclonal sera, hybridoma supernatants or monoclonal antibodies, antibody fragments, Fv, Fab, Fab' and F(ab')2, scFv, DAb or dimers thereof, diabodies, triabodies, tetrabodies and humanized antibodies, and other antibody-like molecules. At the same time, the antibodies may or may not be labelled. Illustrative, but non-exclusive, examples of marker compounds that can be used include radioactive isotopes, enzymes, fluorophores, chemoluminescent reagents, enzyme cofactors or substrates, enzyme inhibitors, particles, dyes, etc.

In a preferred embodiment, the detection of the presence of at least one subunit of PA28 is carried out using antibodies. In a more preferred embodiment, the antibody is a DAb, namely the variable region of an antibody heavy chain (V_{H} domain), selected from the group of DAbs comprising the set of CDR sequences shown in Table 1 or functionally active variants or fragments thereof; said antibodies and their functionally active variants or fragments, constitute an additional aspect of the present invention. According to this aspect, the invention provides a domain antibody which specifically binds to at least one subunit of PA28 and comprises the variable domain of an antibody heavy chain, wherein said domain antibody is selected from the group of domain antibodies fragments listed in Table 1 or functionally active variants or fragments thereof [the skilled person in the art will understand that BGal binds specifically to beta-galactosidase (i.e., it does not bind specifically to at least one subunit of PA28) and it is used as a negative control]. An antibody, or a fragment thereof, which specifically binds to at least one subunit of PA28, comprising said domain antibody also constitutes an additional aspect of the instant invention.

In an even more preferred embodiment, the antibody is the DAb, namely the variable region of an antibody heavy chain (V_{H} domain), comprising a heavy chain CDR1 (HCDR1) with amino acid sequence SEQ ID NO: 13, a HCDR2 with amino acid sequence SEQ ID NO: 14, and a HCDR3 with amino acid sequence SEQ ID NO: 15.

**Table 1**

| **DAbs (variable region of an antibody heavy chain (V_{H} domain))** | | | | | | | | | | |
|---|---|---|---|---|---|---|---|---|---|---|
| Antibody | CDR1 | CDR2 | CDR3 | MW | pI | (D+E) | (R+K) | Cys | I.I. | A.I. |
| 010H01 | GDNLID*T (SEQ ID NO: 1) | IEDNDGST (SEQ ID NO: 2) | AGDWYSYFAVRFADVRDTKVKS (SEQ ID NO: 3) | 13409 | 9.34 | 9 | 13 | 2 | 24.2 | 84.5 |
| 011A10 | GFSVND*F (SEQ ID NO: 4) | IAANNGST (SEQ ID NO:5) | AGEGWSAAGEDTMRS (SEQ ID NO: 6) | 12912 | 4.96 | 11 | 9 | 2 | 28.5 | 69.3 |
| 010D03 | GDRVSYDD (SEQ ID NO: 7) | IENPDGST (SEQ ID NO: 8) | AGNTSDLWDHGWAKRTKEVPF (SEQ ID NO: 9) | 13900 | 5.28 | 14 | 12 | 2 | 34.8 | 69.1 |
| 011D12 | GFTFTA*A (SEQ ID NO: 10) | IRNQNGST (SEQ ID NO: 11) | AAAFG*ASPIGS (SEQ ID NO: 12) | 12506 | 7.98 | 8 | 9 | 2 | 30.2 | 73.6 |
| 011H12 | GYRVITEF (SEQ ID NO: 13) | INTQNGST (SEQ ID NO: 14) | ASSVEEKNALRY (SEQ ID NO: 15) | 12881 | 8.64 | 9 | 11 | 2 | 27.6 | 78.5 |
| i11D12 | GDTFIH*D (SEQ ID NO: 16) | IREQNGST (SEQ ID NO: 17) | ATSVGPAPYHMYF (SEQ ID NO: 18) | 12853 | 6.91 | 9 | 9 | 2 | 31.2 | 72.1 |
| 020D11 | GFMLNY*Y (SEQ ID NO: 19) | IRGDSGST (SEQ ID NO: 20) | ATTSWSGEHHFEY (SEQ ID NO: 21) | 12857 | 9.03 | 9 | 12 | 2 | 36.2 | 75.2 |
| 010G05 | GYKVRP*D (SEQ ID NO: 22) | IEGQGGST (SEQ ID NO: 23) | ARFSLCQDAMGY (SEQ ID NO: 24) | 12900 | 7.88 | 10 | 11 | 3 | 34.2 | 71.3 |
| 010D06 | GYNFSDED (SEQ ID NO: 25) | ICGANGST (SEQ ID NO: 26) | AASKDCMLEEYSMKS (SEQ ID NO: 27) | 13088 | 4.99 | 12 | 10 | 4 | 29.7 | 68.5 |
| 010A05 | RR*AYRSD (SEQ ID NO: 28) | IRTSNGST (SEQ ID NO: 29) | AGVATVTSELTY (SEQ ID NO: 30) | 12925 | 9.3 | 8 | 12 | 2 | 35.9 | 74.5 |
| 020F08 | YAKLSDQN (SEQ ID NO:31) | IRNSNGST (SEQ ID NO: 32) | ARLLVPTLVATTLTF (SEQ ID NO: 33) | 13230 | 9.3 | 7 | 11 | 2 | 30 | 85.5 |
| BGal | GVSITAES (SEQ ID NO: 34) | ITMRDGST (SEQ ID NO: 35) | ARARAMYPLRSSQLES (SEQ ID NO: 36) | 13070 | 9.04 | 8 | 11 | 2 | 38 | 71.7 |

| | | | | | | | | | | |
|---|---|---|---|---|---|---|---|---|---|---|
| MW: estimated molecular weight; pI: isoelectric point; (D+E): total of acid amino acids; (R+K): total of basic amino acids; Cys: number of cysteines; I.I.: instability index; A.I.: aliphatic index. BGal binds specifically to beta-galactosidase and it is used as a negative control. | | | | | | | | | | |

As used herein, the term "CDR" or "complementary determining region" or "hypervariable region" refers to the restricted regions within the variable regions of intact antibodies or antibody fragments that bind to antigenic determinants, i.e. complement an antigen's shape. The CDRs are the most variable part of an antibody and contribute to the diversity of these molecules, allowing the antibody to recognize a vast repertoire of antigens. Thus, the CDRs determine the affinity and specificity of an antibody for specific antigens. Starting from the heavy chain CDRs, the person skilled in the art will be able to obtain a V_{H} (DAb) by cloning the sequences corresponding to said CDRs into the framework of an immunoglobulin heavy chain variable domail.

There is a wide variety of well known assays that can be used in the present invention, using non-labelled antibodies (primary antibody) and labelled antibodies (secondary antibodies); these techniques are based on immunoassays and include, among others, immunohistochemistry and immunofluorescence.

In a particular embodiment of the first diagnostic method of the invention, wherein the detection of the presence of at least one subunit of PA28 is performed by means of a technique selected from the group consisting of immunohistochemistry and immunofluorescence. In a preferred embodiment, the detection of the presence of at least one subunit of PA28 on the cell surface is performed by means of immunohistochemistry.

Immunohistochemistry is a staining method based on immunoenzymatic reactions using monoclonal or polyclonal antibodies to detect cells or specific proteins such as tissue antigens. Typically, immunohistochemistry protocols include detection systems that make the presence of the markers visible (to either the human eye or an automated scanning system), for qualitative or quantitative analyses. Various immunoenzymatic staining methods are known in the art for detecting a protein of interest. For example, immunoenzymatic interactions can be visualized using different enzymes such as peroxidase, alkaline phosphatase, or different chromogens such as DAB, AEC or Fast Red. The method of the first aspect may be accomplished using any suitable method or system of immunohistochemistry, as will be apparent to one skilled in the art, including automated systems, quantitative IHC, semi-quantitative IHC, and manual methods. As used herein, "quantitative" immunohistochemistry refers to an automated method of scanning and scoring samples that have undergone immunohistochemistry, to identify and quantitate the presence of a specified biomarker, such as an antigen or other protein. The score given to the sample is a numerical representation of the intensity of the immunohistochemical staining of the sample, and represents the amount of target biomarker present in the sample. As used herein, Optical Density (OD) is a numerical score that represents intensity of staining. As used herein, semi-quantitative immunohistochemistry refers to scoring of immunohistochemical results by human eye, where a trained operator ranks results numerically (e.g., as 1, 2 or 3). Various automated sample processing, scanning and analysis systems suitable for use with immunohistochemistry are available in the art. Such systems may include automated staining and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. See, e.g., the CAS-200 system (Becton, Dickinson & Co.).

Immunofluorescence is a technique used for light microscopy with a fluorescence microscope and is used primarily on biological samples. This technique uses the specificity of antibodies to their antigen to target fluorescent dyes to specific biomolecule targets within a cell, and therefore allows visualisation of the distribution of the target molecule through the sample. Immunofluorescence is a widely used example of immunostaining and is a specific example of immunohistochemistry that makes use of fluorophores to visualise the location of the antibodies. Immunofluorescence can be used on tissue sections, or individual cells, and may be used to analyse the distribution of PA28 and/or any or both of its subunits PSME1 and PSME2. Immunofluoresence can be used in combination with other, non-antibody methods of fluorescent staining, for example, use of DAPI to label DNA. Preferred dyes to be used in combination with PA28 and/or any or both of its subunits PSME1 and PSME2 are DAPI and/or AE1/AE3. Several microscope designs can be used for analysis of immunofluorescence samples; the simplest is the epifluorescence microscope, and the confocal microscope is also widely used. Various super-resolution microscope designs that are capable of much higher resolution can also be used.

There are two classes of immunofluorescence techniques, primary (or direct) and secondary (or indirect). Primary, or direct, immunofluorescence uses a single antibody that is chemically linked to a fluorophore. The antibody recognizes the target molecule and binds to it, and the fluorophore it carries can be detected via microscopy. This technique has several advantages over the secondary (or indirect) protocol below because of the direct conjugation of the antibody to the fluorophore. This reduces the number of steps in the staining procedure making the process faster and can reduce background signal by avoiding some issues with antibody cross-reactivity or non-specificity. However, since the number of fluorescent molecules that can be bound to the primary antibody is limited, direct immunofluorescence is less sensitive than indirect immunofluorescence. Secondary, or indirect, immunofluorescence uses two antibodies; the unlabeled first (primary) antibody specifically binds the target molecule, and the secondary antibody, which carries the fluorophore, recognises the primary antibody and binds to it. Multiple secondary antibodies can bind a single primary antibody. This provides signal amplification by increasing the number of fluorophore molecules per antigen. This protocol is more complex and time consuming than the primary (or direct) protocol above, but it allows more flexibility because a variety of different secondary antibodies and detection techniques can be used for a given primary antibody.

Analysis by means of immunohistochemistry and immunofluorescence requires the use of a specific antibody for the target proteins PA28 and/or any or both of its subunits PSME1 and PSME2. Different primary antibodies with different constant regions are typically generated by raising the antibody in different species. For example, a researcher might create primary antibodies in a goat that recognize several antigens, and then employ dye-coupled rabbit secondary antibodies that recognize the goat antibody constant region ("rabbit anti-goat" antibodies). The researcher may then create a second set of primary antibodies in a mouse that could be recognized by a separate "donkey anti-mouse" secondary antibody. This allows re-use of the difficult-to-make dye-coupled antibodies in multiple experiments. Alternatively, commercially available antibodies may be used, such as those provided by Santa Cruz Biotech., Cell Signalling, Thermo, etc.

In a preferred embodiment, the detection of the presence of at least one subunit of PA28 is carried out using antibodies. In a more preferred embodiment, the antibody is a DAb selected from the group of DAbs listed in Table 1 or functionally active variants or fragments thereof.

In an even more preferred embodiment, the antibody is the DAb, namely the variable region of an antibody heavy chain (V_{H} domain), comprising a heavy chain CDR1 (HCDR1) with amino acid sequence SEQ ID NO: 13, a HCDR2 with amino acid sequence SEQ ID NO:14, and a HCDR3 with amino acid sequence SEQ ID NO: 15.

The term "prostate tissue sample", as used herein, refers to any tissue sample obtained from the prostate. Preferably, the sample will contain tumour cells. In a particular embodiment, the prostate tissue sample is a biopsy. Virtually, any conventional method can be used within the frame of the invention to obtain a prostate tissue sample from a subject.

Once the at least one subunit of PA28 has been detected in a prostate tissue sample, the person skilled in the art will be able to determine whether it is located on the cell surface or not. The presence of at least one subunit of PA28 on the cell surface is indicative of the subject from which the prostate tissue sample was taken suffering from prostate cancer. Thus, in a second step of the first diagnostic method of the invention, the subject is diagnosed as having prostate cancer if the presence of at least one subunit of PA28 on the cell surface is detected.

In a particular embodiment, the first diagnostic method of the invention comprises detecting the presence of one subunit of PA28 in a prostate tissue sample from said subject, wherein the detection of said one subunit of PA28 on the cell surface is indicative of said subject having prostate cancer.

In a preferred embodiment of the first diagnostic method of the invention, the PA28 subunit is selected from the group consisting of PSME1 and PSME2. In a most preferred embodiment, the subunit is PSME1.

The authors of the present invention have also demonstrated that PA28 is overexpressed in prostate cancer cells compared to normal prostate cells. This finding can also be exploited in the diagnosis of prostate cancer in a subject. Therefore , in a another aspect, the present invention relates to *an in vitro* method for diagnosing prostate cancer in a subject, hereinafter "second diagnostic method of the invention", comprising quantifying the expression level of at least one subunit of PA28 in a prostate tissue sample from said subject and comparing the level of said at least one subunit of PA28 in the sample with a reference value, wherein a level of said at least one subunit of PA28 in said sample higher than a reference value is indicative of said subject having prostate cancer.

The terms "method for diagnosing", "prostate cancer", "subject", "prostate tissue sample", and "PA28" and subunits thereof have been described in detail above and are equally applied to the second diagnostic method of the invention

In a first step, the second diagnostic method of the invention comprises quantifying the expression level of at least one subunit of PA28 in a prostate tissue sample from said subject. By way of a non-limiting illustration, the expression levels are determined by means of the quantification of the protein levels or of the levels of mRNA that encode said proteins.

As used herein, "expression level" refers to the level at which a protein, e.g. the at least one subunit of PA28, is expressed in a cell. The methods for quantifying the expression level of a molecule are well-known by a person skilled in the art and include a number of alternatives. These can be at the protein level or at the nucleic acid level. Virtually any method which allows quantifying the expression level of at least one subunit of PA28 can be used in putting the second method of the invention into practice.

By way of non-limiting illustration, "protein level" refers but is not limited to a quantifiable, semiquantifiable, or relative amount of the at least one subunit of PA28, as well as to any other value or parameter related to said protein or which can be derived therefrom. Said values or parameters comprise signal intensity values obtained from any of the physical or chemical properties of said protein obtained by means of direct measurement or by means of indirect measurement.

The quantification of the expression level of at least one subunit of PA28 at the protein level can be carried out using any conventional method. By way of a non-limiting example, said detection can be carried out using antibodies with the capacity for binding specifically to the protein to be detected (or to fragments thereof containing the antigenic determinants) and subsequent detection of the resulting antigen-antibody complexes.

The term "antibody" has have been described in detail above and the term and all its particulars are equally applied to the second diagnostic method of the invention.

The antibodies that are going to be used in this type of assay can be, for example, polyclonal sera, hybridoma supernatants or monoclonal antibodies, antibody fragments, Fv, Fab, Fab' and F(ab')2, scFv, DAb or dimers thereof, diabodies, triabodies, tetrabodies and humanized antibodies, and antibody-like molecules. At the same time, the antibodies may or may not be labelled. Illustrative, but non-exclusive, examples of marker compounds that can be used include radioactive isotopes, enzymes, fluorophores, chemoluminescent reagents, enzyme cofactors or substrates, enzyme inhibitors, particles, dyes, etc. There is a wide variety of well known assays that can be used in the present invention, using non-labelled antibodies (primary antibody) and labelled antibodies (secondary antibodies); these techniques include immunocytochemical and immunohistochemical techniques.

In a preferred embodiment, the quantification of the expression level of at least one subunit of PA28 is carried out using antibodies. In a more preferred embodiment, the antibody is a DAb selected from the group of DAbs listed in Table 1 or functionally active variants or fragments thereof.

In an even more preferred embodiment, the antibody is the DAb, namely the variable region of an antibody heavy chain (V_{H} domain), comprising a heavy chain CDR1 (HCDR1) with amino acid sequence SEQ ID NO: 13, a HCDR2 with amino acid sequence SEQ ID NO: 14, and a HCDR3 with amino acid sequence SEQ ID NO: 15.

In a particular embodiment of the second diagnostic method of the invention, wherein quantification of the level of at least one subunit of PA28 is performed by means of a technique selected from the group consisting of immunohistochemistry and immunofluorescence. In a preferred embodiment, the quantification of the level of at least one subunit of PA28 is performed by means of immunohistochemistry. Immunohistochemistry and immunofluorescece techniques have been described in detail in relation with the first diagnostic method of the invention and their particulars are equally applied to the second diagnostic method of the invention.

The expression level of the at least one subunit of PA28 can also be quantified at the nucleic acid level. Virtually any conventional method can be used within the frame of the invention to detect and quantify the mRNA levels of said marker proteins.

Methods for determining the quantity of mRNA are well known in the art. For example the nucleic acid contained in the sample (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e.g., Northern blot analysis, *in situ* hybridization assays, or by oligonucleotide microarrays after converting the mRNA into a labeled cDNA) and/or amplification (e.g., RT-PCR). Preferably quantitative or semi-quantitative RT-PCR is preferred. Real-time quantitative or semi-quantitative RT-PCR is particularly advantageous. Preferably, primer pairs were designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA). Preferably, the quantity of mRNA is measured by *in situ* hybridization assays, quantitative or semi-quantitative RT-PCR or by real-time quantitative or semi-quantitative RT-PCR.

Once the level of at least one subunit of PA28 in a prostate tissue sample from a subject has been determined, the level is then compared with reference values for each of said proteins. Typically, reference values are the levels of the proteins being compared in a reference sample, in this case the at least one subunit of PA28.

A "reference value", as used herein, generally refers to the level of a product, for example, the at least one subunit of PA28, present in control subjects. In a particular embodiment, said control subjects are subjects who do not suffer a specific disease (e.g., prostate cancer), whereas in another particular embodiment, said control subject is the actual subject under study, which is particularly useful for evaluating the tracking of a disease (e.g., prostate cancer) or for evaluating the efficacy of a treatment for said disease (e.g., prostate cancer), etc., for which the reference level of a given product can be the level of said product determined in a sample from the same subject under study but taken days, weeks, months or even years before for the purpose of evaluating the tracking of the disease, or taken before, for example, the application in the subject of a treatment for said disease for the purpose of evaluating its efficacy.

Due to the variability that can occur among the different subjects in terms of the production of the at least one subunit of PA28, the reference level could be obtained from a set of samples from a population of healthy subjects (e.g., subjects who do not suffer prostate cancer) and by calculating the mean level of the product in question (the at least one subunit of PA28) in said population of healthy subjects.

The reference level of a certain product, for example, the at least one subunit of PA28, can be determined from a reference sample which can be analyzed, for example, simultaneously or consecutively, together with the sample to be analyzed (test sample). The reference level can generally be derived from the normal distribution limits of a physiological amount found in a population of control subjects. Said physiological amount can be determined by several well-known techniques, as described in this description.

According to the present invention, said reference level allows discriminating the presence of the at least one subunit of PA28 and can therefore be used in the diagnosis of prostate cancer

Once level of at least one subunit of PA28 in relation to reference values for said proteins or nucleic acids have been determined, it is necessary to identify if there are alterations in the expression of said proteins or nucleic acids (increase or decrease of the expression). The expression of a protein or nucleic acid is considered increased in a sample of the subject under study when the levels increase with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Similarly, the expression of a protein or nucleic acid is considered decreased when its levels decrease with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

Lastly, if the expression level of the at least one subunit of PA28 in a prostate tissue sample from said subject is higher than a reference value, it is indicative of said subject having prostate cancer. Thus, in a last step of the second diagnostic method of the invention, the subject is diagnosed as having prostate cancer if the expression level of the at least one subunit of PA28 in a prostate tissue sample from said subject is higher than a reference value.

In a particular embodiment of the second diagnostic method of the invention, the method comprises quantifying the expression level of one subunit of PA28 in a prostate tissue sample from said subject and comparing the level of said one subunit of PA28 in the sample with a reference value, wherein a level of said one subunit of PA28 in said sample higher than a reference value is indicative of said subject having prostate cancer.

In another particular embodiment, the PA28 subunit is selected from the group consisting of PSME1 and PSME2. In a more preferred embodiment, the PA28 subunit is PSME1.

In another particular embodiment of the second diagnostic method of the invention, the subject is a mammal and includes, but is not limited to, domestic and farm animals, primates and humans, for example, human beings, non-human primates, cows, horses, pigs, sheep, goats, dogs, cats, or rodents. In a preferred embodiment, the subject is a male human being of any age or race.

In another particular embodiment of the second diagnostic method of the invention, the prostate tissue sample will contain tumour cells. In a particular embodiment, the prostate tissue sample is a biopsy. Virtually, any conventional method can be used within the frame of the invention to obtain a prostate tissue sample from a subject.

The presence or the increased level of PA28 or any subunit thereof in blood from a subject is also an indication that the subject suffers from prostate cancer. Thus, in a another aspect, the present invention relates to *an in vitro* method for diagnosing prostate cancer in a subject, hereinafter "third method of the invention", comprising quantifying the expression level of at least one subunit of PA28 in a biological fluid sample from said subject, and comparing said level of at least one subunit of PA28 with a reference value, wherein a level of said at least one subunit of PA28 in said sample higher than a reference value is indicative of said subject having prostate cancer.

The terms "method for diagnosing", "prostate cancer", "subject", "PA28" and subunits thereof, "quantifying the expression level" and "reference value" have been described in detail above and are equally applied to the third diagnostic method of the invention.

In a first step, the third diagnostic method of the invention comprises quantifying the expression level of at least one subunit of PA28 in a biological fluid sample from a subject.

As used herein, the term "biological fluid sample" and "biofluid" are used interchangeably herein and refer to aqueous fluids of biological origin. The biofluid may be obtained from any location (such as blood, plasma, serum, urine, bile, cerebrospinal fluid, aqueous or vitreous humour, or any bodily secretion), an exudate (such as fluid obtained from an abscess or any other site of infection or inflammation), or ejaculate.

In a particular embodiment of the third diagnostic method of the invention, the biofluid is selected from the group consisting of blood, plasma, serum, urine and ejaculate. In a preferred embodiment the biofluid is serum or urine.

The methods for quantifying the protein level (concentration) are well-known by a person skilled in the art and include a number of alternatives. Virtually any method which allows quantifying the expression level of at least one subunit of PA28 can be used in putting the third method of the invention into practice, and these may be at the protein or at the nucleic acid level.

The quantification of the protein level of at least one subunit of PA28 can be carried out using any conventional method. By way of a non-limiting example, said detection can be carried out using intact antibodies, antibody fragments containing the antigenic determinants, or antibody-like molecules with the capacity for binding specifically to the protein to be detected and subsequent detection of the resulting antigen-antibody complexes.

The antibodies that are going to be used in this type of assay have been described in detail in relation with the first and second diagnostic methods of the invention and the term and its particulars are equally applied to the third diagnostic method of the invention.

Methods suitable for the quantification of the expression level of at least one subunit of PA28 at the protein level have been described in detail in relation with the first and second diagnostic methods of the invention and are equally applied to the third diagnostic method of the invention. Similarly, methods suitable for the quantification of the expression level of at least one subunit of PA28 at the nucleic level have been described in detail in relation with the second diagnostic method of the invention and are equally applied to the third diagnostic method of the invention.

Additionally, there is a wide variety of well known assays that can be used in the third diagnostic method of the invention, using non-labeled antibodies (primary antibody) and labeled antibodies (secondary antibodies); these techniques include Western-blot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, techniques based on the use of biochips or protein microarrays including specific antibodies or assays based on the colloidal precipitation in formats such as reagent strips. Other forms of detecting and quantifying the protein include affinity chromatography techniques, ligand-binding assays, etc.

Once the expression level of at least one subunit of PA28 in a biofluid sample from a subject has been determined, the expression level is then compared with reference values for each of said proteins. Typically, reference values are the expression levels of the proteins being compared in a reference sample, in this case the at least one subunit of PA28.

Once the expression level of at least one subunit of PA28 in relation to reference values for said proteins have been determined, it is necessary to identify if there are alterations in the expression of said proteins (increase or decrease of the expression). The expression of a protein is considered increased in a biofluid sample of the subject under study when the levels increase with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Similarly, the expression of a protein is considered decreased when its levels decrease with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

Lastly, if a level of the at least one subunit of PA28 in a biofluid sample from said subject is higher than a reference value, it is indicative of said subject having prostate cancer.

In a particular embodiment of the third diagnostic method of the invention, the method comprises quantifying the expression level of one subunit of PA28 in a biofluid sample from said subject and comparing the level of said one subunit of PA28 in the sample with a reference value, wherein a level of said one subunit of PA28 in said sample higher than a reference value is indicative of said subject having prostate cancer.

In a preferred embodiment, the PA28 subunit is selected from the group consisting of PSME1 and PSME2. In a more preferred embodiment, the PA28 subunit is PSME1.

In another particular embodiment of the second diagnostic method of the invention, the subject is a mammal and includes, but is not limited to, domestic and farm animals, primates and humans, for example, human beings, non-human primates, cows, horses, pigs, sheep, goats, dogs, cats, or rodents. In a preferred embodiment, the subject is a male human being of any age or race.

It will be understood that the method according to the present invention may comprise the determination of any naturally occurring isoforms of one or more of the above proteins.

### Therapeutic agents of the invention

In a another aspect, the present invention relates to a therapeutic agent that binds specifically to at least one subunit of PA28 for use in the treatment of a subject suffering from prostate cancer, hereinafter "therapeutic agent of the invention", wherein said therapeutic agent comprises a first component selected from the group consisting of
- an intact antibody that binds specifically to at least one subunit of PA28;
- an antibody fragment that binds specifically to at least one subunit of PA28; and
- an antibody-like molecule that binds specifically to at least one subunit of PA28.

The terms, "prostate cancer", "subject", "PA28" and subunits thereof, "intact antibody", "antibody fragment", and "antibody-like molecule" have been described in detail in relation with the diagnostic methods of the invention, and the terms and their particulars are equally applied to the third diagnostic method of the invention.

As used herein, the term "therapeutic agent" refers to an agent suitable for being used in the treatment of prostate cancer.

In a particular embodiment, the first component of the therapeutic agent of the invention comprises an intact antibody that binds specifically to at least one subunit of PA28.

In another particular embodiment, the therapeutic agent of the invention comprises an antibody fragment that binds specifically to at least one subunit of PA28.

In another particular embodiment, the therapeutic agent of the invention comprises an antibody-like molecule that binds specifically to at least one subunit of PA28.

In a particular embodiment, the therapeutic agent of the invention binds specifically to one subunit of PA28 selected from the group consisting of PSME1 and PSME2. In a preferred embodiment, the PA28 subunit is PSME.

The term "treatment" includes any process, action, application, therapy, or the like, wherein a subject (or patient), including a human being, is provided medical aid with the object of improving the subject's condition, directly or indirectly, or slowing the progression of a condition or disorder in the subject, or ameliorating at least one symptom of the disease or disorder under treatment.

In another particular embodiment, the therapeutic agent of the invention further comprises a second component selected from the group consisting of:
- a radionuclide, including but not limited to ¹³I, ⁹⁰Y, ¹⁷⁷Lu, etc.;
- a toxin, including but not limited to ricin, Pseudomonas exotoxin A, saporin, gelonin, etc.;
- an enzyme, including but not limited to glycosidases (β-glucuronidase, β-glucosidase, β-galactosidase), β -lactamase, cellulase, dextranase, fructase, aminopeptidase, lysozyme, cytosine deaminase, carboxypeptidase, penicillin amidase, methionine γ liase, carboxyesterase, etc.;
- a cytokine, including interleukins, such as IL-2, IL-4, IL-6, IL-7, IL-10, IL-12, IL-17, etc.; interferons, such as IFN-γ, etc.; tumour growth factors, such as TGF-α, TGF-β, etc.;
- an angiogenesis inhibitor, including but not limited to endostatin, angiostatin, restin, etc.

It will be understood by the skilled person that the first and the second components comprising the therapeutic agent of the invention may constitute a fusion protein or a protein conjugate.

The present invention contemplates different methods for the addition of the second component to the first component according to the invention, including the generation of fusion proteins and protein conjugates.

As used herein, the term "fusion protein" or "chimeric protein" refers to the expression product of a gene encoding for a polypeptide comprising the protein comprising a catalytic domain linked by a covalent, peptide (amide) bond to at least one different protein domain comprising a target binding region. The fusion of at least two protein entities is such that one is allowed to move with respect to the other, and that it does not cause a substantial loss in the activity of one or all the protein entities.

The skilled person will understand that it may be desirable that the fusion protein further comprises a flexible peptide that binds the protein comprising a catalytic domain and the moiety comprising a target binding region. The flexible peptide will permit the movement of one protein with respect to the other.

Therefore, in another particular embodiment, the fusion protein comprising a catalytic domain and a protein domain comprising a target binding region further comprises a flexible peptide.

As used herein, the term "flexible peptide", "spacer peptide" or "linker peptide" refers to a peptide that covalently binds the protein comprising a catalytic domain and the moiety comprising a target binding region, which is not part of neither the protein nor the moiety, allowing movement of one with respect to the other, without causing a substantial detrimental effect on the function of either the protein or the moiety. In a preferred embodiment, said flexible peptide binds the protein and the moiety substantially without causing a detrimental effect on the function of neither the protein nor the moiety. It is not necessary that the protein and the moiety are arranged in that order and, in this case, the invention contemplates fusion proteins in which the protein is located at amino-terminal position relative to the moiety, and wherein the protein is located at carboxyl-terminal position relative to the moiety.

The flexible peptide comprises at least one amino acid, at least two amino acids, at least three amino acids, at least four amino acids, at least five amino acids, at least six amino acids, at least seven amino acids, at least eight amino acids, at least nine amino acids, the least 10 amino acids, at least 12 amino acids, at least 14 amino acids, at least 16 amino acids, at least 18 amino acids, at least 20 amino acids, at least 25 amino acids, at least 30 amino acids, at least 35 amino acids, at least 40 amino acids, the least 45 amino acids, at least 50 amino acids, at least 60 amino acids, at least 70 amino acids, at least 80 amino acids, at least 90 amino acids, or about 100 amino acids.

Flexible peptides suitable for use in the present invention are those which have been previously described as suitable for linking two polypeptide domains that allow said polypeptide domains to substantially retain their native structure and activity, such as those disclosed in WO2009150284. In a preferred embodiment, the peptide linker is made up mainly of the residues glycine, serine and/or proline. Peptide linkers suitable for use in the invention include peptides comprising the sequences (GlySer)ₙ, (GlyₘSer)ₙ or (SerₘGly)ₙ, wherein m is 1 to 6, particularly 1 to 4, and typically 2 to 4, n is 1 to 30 or 1 to 10 and typically 1 to 4 and, optionally, comprise some residues of glutamic acid (Glu) or lysine (Lys) distributed along the sequence to enhance solubility (see, for example, WO 96/06641, which provides exemplary linkers for use in conjugates).

In yet another particular embodiment, the therapeutic agent of the invention is a protein conjugate. As used herein, the term "protein conjugate" refers to the product resulting from the chemical joining of the first component and the second components comprised in the therapeutic agent by a non-peptidic, chemical bond. This chemical bond is typically achieved by crosslinking. As used herein, the term "crosslinking", "conjugation" or "chemical conjugation" refers to the process of chemically joining two or more components by a covalent, non-peptidic, bond.

Methods for protein conjugation are well known in the art. For example, protein conjugation can be carried out directly or through a linker moiety, through one or more non-functional groups on the targeting protein and/or the enzyme, such as amine, carboxyl, phenyl, thiol or hydroxyl groups. Conventional linkers can be used, such as diisiocyanates, diisothiocyanates, bis(hydroxysuccinimide) esters, carbodiimides, maleimide-hydroxysuccinimide esters, glutaraldehyde and the like, or hydrazines and hydrazides, such as 4-(4-N-maleimidophenyl)butyric acid hydrazide (MPBH).

A simple method for making a protein:protein conjugate is to mix the two proteins in the presence of glutaraldehyde to form the conjugate. The initial Schiff base linkages can be stabilized, e.g., by borohydride reduction to secondary amines. A diisothiocyanate or a carbodiimide can be used in place of glutaraldehyde.

More selective linkage can be achieved by using a heterobifunctional linker such as a maleimide-hydroxysuccinimide ester. Reaction of the latter with an enzyme will derivatize amine groups on, e.g., an enzyme, and the derivative can then be reacted with, e.g., an antibody fragment with free sulfhydryl groups.

It is advantageous to link protein comprising a catalytic domain to a site on the targeting protein remote from the binding site. Non-limiting examples of methods to accomplish this include linkage to cleaved interchain sulfhydryl groups, as noted above. Another method involves reacting, e.g. an antibody whose carbohydrate portion has been oxidized with an enzyme which has at least one free amine function. This results in an initial Schiff base (imine) linkage, which is preferably stabilized by reduction to a secondary amine, e.g., by borohydride reduction, to form the final conjugate. See also the discussion above of the methods described in U.S. Pat. No. 5,772,981 and hydrazine and hydrazide linkages.

In a preferred embodiment, the therapeutic agent of the invention consists of a fusion protein comprising a second component, wherein said second component consists of an enzyme. As the person skilled in the art will be able to appreciate, said fusion protein comprising an antibody and an enzyme is useful in the field of targeted therapies, particularly for antibody-directed enzyme prodrug therapy or ADEPT.

Firstly described in 1987 (Bagshawe, 1987, Br J Cancer 56:531-2.), ADEPT is a well-established technique that aims to restrict the cytotoxic action to tumor sites. In brief, an enzyme is coupled to an intact antibody (or another binding region, i.e., antibody fragment or antibody-like molecule) with affinity to tumor-associated antigens. This fusion protein is systematically delivered to its site of action: a tumor tissue. After clearance of unbound enzyme-antibody complexes in the bloodstream, a non-toxic prodrug is administered intravenously and converts into a highly cytotoxic drug upon contact with its activator - the antibody-bound enzyme in the tumor. Selectivity is thus achieved in two respects: in the first place by the highly specific tumor binding of the antibody and, secondly, by delaying prodrug administration in order to allow for a sufficient discrimination between tumor and normal tissue enzyme levels.

### Kits of the invention

The present invention also provides kits for carrying out the above-described methods.

Thus, in another aspect, the invention relates to a kit for diagnosing prostate cancer, hereinafter "first kit of the invention", comprising at least a reagent for performing any of the diagnostic methods of the invention.

As it will be understood by the skilled person, the diagnostic methods of the invention comprise a step of:
- detecting the presence of at least one subunit of PA28, or
- quantifying the expression level of at least one subunit of PA28.

The detection step of the first method of the invention is carried out at the protein level, whereas the quantification step of the second and third methods of the invention may be carried out at the protein level or at the nucleic acid level. Therefore, the reagents suitable for performing any of the diagnostic methods of the invention include:
- antibodies specific for at least one subunit of PA28, and/or
- reagents specific for the nucleic acid (mRNA) encoding the at least one subunit of PA28.

The antibodies specific for at least one subunit of PA28 have been described in detail in the context of the diagnostic methods of the invention, and the terms and their particulars are equally applied to the first kit of the invention.

The reagents specific for the nucleic acid (mRNA) encoding the at least one subunit of PA28 include probes which are capable of specifically hybridizing to said nucleic acid.

In a particular embodiment, the first kit of the invention comprises an intact antibody that binds specifically to at least one subunit of PA28.

In another particular embodiment, the first kit of the invention comprises an antibody fragment that binds specifically to at least one subunit of PA28.

In another particular embodiment, the first kit of the invention comprises an antibody-like molecule that binds specifically to at least one subunit of PA28.

In a particular embodiment, the therapeutic agent of the invention binds specifically to one subunit of PA28 selected from the group consisting of PSME1 and PSME2. In a preferred embodiment, the PA28 subunit is PSME1.

In another aspect, the invention relates to the use of the first kit of the invention for diagnosing prostate cancer.

In another aspect, the present invention relates to a kit comprising the therapeutic agent of the invention, hereinafter "second kit of the invention".

The terms, "prostate cancer", "diagnosing", and "therapeutic agent" have been described in detail above and their particulars are equally applied to the kits of the invention.

In another aspect, the invention relates to the use of the second kit of the invention for the treatment of prostate cancer.

In a particular embodiment, the first and second kits of the invention comprise an antibody, antibody fragment or antibody-like molecule of the invention, with specificity for at least one subunit of PA28. In a more preferred embodiment, the antibody is a V_{H} (DAb) selected from the group consisting of the V_{H} comprising a set of CDR sequences as shown in Table 1 or functionally active variants thereof.

In an even more preferred embodiment, the antibody is the V_{H} (DAb) comprising a heavy chain CDR 1 (HCDR1) with amino acid sequence SEQ ID NO: 13, a HCDR2 with amino acid sequence SEQ ID NO: 14, and a HCDR3 with amino acid sequence SEQ ID NO: 15.

In the context of the present invention, "kit" is understood as a product containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

### Further aspects of the invention

In a further aspect, the invention relates to PA28 or a subunit thereof as a prostate cancer marker.

The terms "PA28", the subunits thereof and "prostate cancer" have been described in detail above and their particulars are equally applied to the kits of the invention.

As used herein, the term "marker" or "tumour marker" refers to a protein that is elevated in cancer in comparison with other healthy tissue samples.

The invention is described below by means of the following examples which are merely illustrative and by no means limiting for the scope of the invention.

### EXAMPLE 1

### Identification of proteasome activator complex PA28 as a target in prostate cancer by in vivo selection of human antibodies

### 1. MATERIALS AND METHODS

### Ethics Statement

All experimental procedures were performed in accordance with the Spanish Government Guidelines for the Care and Use of Laboratory Animals and were approved by the Hospital Universitario Puerta de Hierro Animal Care and Use Committee (CEBA).

### Tumour models

Wild-type human prostatic adenocarcinoma PPC-1 cells (PC-3, CRL-1435) were obtained from the American Type Culture Collection (Rockville, MD, USA) and were cultured in DMEM (Dulbecco's Modified Eagle Medium) supplemented with heat-inactivated 10% (vol/vol) FBS (fetal bovine serum), 2mM L-glutamine, 100 UI/ml penicillin, 0.1 mg/ml streptomycin (all from Life Technologies, Carlsbad, CA, USA). 2 x 10⁶ cells in PBS (phosphate buffered saline) were inoculated subcutaneously in one flank in five-week-old male Hsd: Athymic Nude-Foxn1^{nu} mice (Harlan Ibérica, Barcelona, Spain). Mice were kept under anesthesia during all manipulations and all efforts were made to minimize suffering.

### Phage library and bacterial strains

DAb library was from Source BioScience LifeSciences (Nottingham, UK). This library is based on a single V_{H} framework (V3-23/D47) with diversity (about 3 x 10⁹) introduced into CDR1, CDR2 and CDR3 (Christ et al., 2006, Nucleic Acids Res 34:e108). The phage library was propagated and purified as described (Sanchez-Martin et al., 2011, J Biol Chem. 286:5197-203) using the helper phage KM13. Phage were rescued by infection of exponentially growing E. coli TG1Tr for 1 h at 37 °C, and plated in LB supplemented with 0.1 mg/ml ampicillin and 4% glucose. To learn the CDR composition and abundance of the antibodies, single colonies were analyzed as described (Sanchez-Martin et al., 2011 cited *supra)* after each round of selection.

### Ex vivo selections

Tumours were harvested at a volume of 0.3 - 0.6 cm³ following an intracardial perfusion with pre-warmed DMEM supplemented with 1% BSA (bovine serum albumin). Tumours were rinsed, weighed, minced and disaggregated using a tissue homogenizer (Beckton Dickinson, Bedford, MA, USA) to obtain a 30 mg/ml suspension. 1 ml of this suspension was mixed with 9 ml containing 1 x 10¹² TU for 16 h in continuous rotation at 4 °C. The suspension was centrifuged, the pellet was washed 5 times in 10 ml and resuspended in 0.5 ml of PBS containing 1 mg/ml trypsin at room temperature for 15 min. Trypsin was neutralized by adding FBS to a final 0.5% and cellular debris was discarded by centrifugation (14000 rpm, for 10 min in a bench centrifuge). Phages were rescued as above.

### In vivo selections

The preselected repertoire (0.1 ml of PBS containing 1 x 10¹² TU) was injected into the tail vein of tumour-bearing mice when the tumour reached a volume of 0.3 - 0.6 cm³. After the specified circulation time (from 15 min to 24 h) the mice were perfused as above, and organs (tumour, kidney, heart, a fragment of the liver) were collected, rinsed with PBS, weighed and homogenized in 2 ml of PBS supplemented with 1% NP-40 and 1 mg/ml trypsin using an OmniTH handheld homogenizer (Omni International, Kennesaw, GA, USA). After 30 min of incubation at room temperature the recovered phage were rescued as above.

Two parameters were calculated: the total phage number obtained per milligram of tissue, and a normalized value -calculated as the ratio between the TU per mg of tissue and the average TU per mg for the organs of reference- that allows the comparison of different mice and doses.

### Homing assays

A fresh preparation of monoclonal phage (1 x 10¹¹ TU in 0.1 ml PBS) was used for the homing studies, in which the recovered organs were split in two for titration, and frozen for immunofluorescence studies. The total phage number and the normalized value were calculated as above. Sections were fixed with 4% paraformaldehyde, permeabilized using 0.1% Triton X100, and stained with the primary antibodies at 0.010 mg/ml. As secondary antibodies, a 1:1000 dilution of Alexa-conjugated polyclonal antibodies were used. Nuclei were stained using To-Pro-3 iodide (Life Technologies) according to the manufacturer's instructions. Sections were mounted using a DAPI-containing medium (Vector Labs, Burlingame, CA, USA). The antibodies used were from Sigma-Aldrich (St Louis, MO, USA): rabbit-anti-Fd; AbCam (Cambridge, UK): mouse polyclonal to PSME1, mouse isotype control IgG1; BD Pharmingen (San Jose, CA, USA): rat mAb anti-CD31; and Life Technologies: goat anti-rat IgG A546, goat anti-rabbit IgG A546, goat anti-rabbit IgG A488, goat anti-mouse IgG A488, and goat anti-mouse IgG A546. Quantification of the total area positive for anti-CD31 or anti-phage staining was calculated on images acquired with a Nikon Eclipse Ti (Nikon Corp., Tokyo, Japan) microscope. Ten non-overlapping random fields, excluding the rim of the tumour, were acquired for each section with the appropriate filters (Alexa-488 and Alexa-546) and were analyzed using the ImageJ software (Abramoff et al., 2004, Biophotonics International 11:36-42.) A relative parameter (V, calculated as the total area positive for the phage staining divided by the total area positive for the CD31 staining) aims to correlate the amount of phage with vessel availability (CD31 staining) per field.

### Statistical analysis of the composition of the CDR's

The frequencies of all possible combinations of three residues within the CDR's were calculated using a Python (version 3.2) module that yields a multidimensional matrix with the position of each residue, composition, and number of cases for the sequences recovered. Because the interaction between the antibody and its ligand may involve non-contiguous amino acids from the different CDR's (and because the framework is constant), only the amino acids of the CDR's were considered. The selectivity of the three residues in that position was assessed by a Fisher's exact test and calculated considering its count in the tumour as compared to the combined count in the other organs. Statistical significance was considered for p-values lower than 0.05, and was further assessed by a resampling simulation (Kolonin et al., 2006, FASEB J. 20:979-81). Sequences from the tumour and the organs were pooled and resampled into 1000 permutated groups (corresponding in size to those analyzed previously) that were later re-evaluated for the multidimensional matrix. Fisher's exact test was performed in the 1000 randomized datasets, and distributions of the 50 lowest p-values generated in each simulated test were graphically compared with the distribution of the 50 lowest p-values from the experimental data. For each of the plotted p-values, simulation-based statistical was evaluated as the number of smaller p-values than the experimental divided by the total number of simulations.

The same approach was followed with the combined output from all the *in vivo* rounds of selection and those combinations of position and residues that were preferentially (experimental p-value < 0.05) accumulated in the tumour were used to calculate consensus sequences either absent or present in the tumour.

### Construction of expression vectors and purification of antibodies

The coding sequence of 011H12 was optimized for bacterial expression of the monovalent antibody (GeneArt AG, BioPark Regensburg, Germany). The coding sequences of the anti-BGal and the optimized 011H12 DAb were digested with NcoI/NotI and inserted into the pAB1 plasmid, and induced as described (Sánchez-Martin et al., 2011 cited *supra).* Purification was performed using the AKTAprime plus system (affinity step: HisTrap or HiTrap *rProtein A FF columns (GE Healthcare, Uppsala, Sweden) according to manufacturer's protocol and checked by SDS-PAGE and Western blotting.

The 011H12 DAb and CEA1 VHH coding regions were amplified by PCR using primer pairs ClaI-H12 [5'- TCA TCG ATG GAG GTG CAG CTG GTG GAG TCT -3' (SEQ ID NO: 37)] and FdSeql (Sanchez-Martin et al., 2011 cited *supra)* or ClaI-CEA1 [5'-CAA CGC GTC ATG GCT CAG GTG CAG CTG GT-3' (SEQ ID NO: 38)] and NotI-CEA1 [5'-TGG TGG GCG GCC GCT GAG GAG ACG GTG AC-3' (SEQ ID NO: 39)], and ligated into pCR2.1 TOPO (Life Technologies, Carlsbad, CA, USA). The ClaI/NotI-digested fragments were ligated into the ClaI/NotI digested backbone of the pCR3.1-L36-NC1^{ES}- digested plasmid (Cuesta AM et al. In vivo tumor targeting and imaging with engineered trivalent antibody fragments containing collagen-derived sequences. PLoS ONE. 2009;4:e5381) to obtain the pCR3.1-011H12-NC1^{ES} and pCR3.1-CEA1-NC1^{ES-}. All constructs were verified by sequencing. Human 293T cells (CRL-11268) were transfected using Superfect (QIAGEN GmbH, Hilden, Germany). Supernatant was collected at 48 h and analyzed for antibody expression by Western blotting using anti-myc mAb and ELISA.

### Protein identification by nanoLC-MS

Monoclonal DAbs were purified as described (Sanchez-Martin et al., 2011 cited *supra)* and coupled to the HiTrap NHS-activated HP Columns (GE Healthcare, Uppsala, Sweden) according to the manufacturer's instructions. A fresh tumour lysate was circulated through the coupled columns (first control, then test) so that the first one would remove unespecifically bound molecules, whereas the second could trap the antigen if present in the tumour lysate. After the circulation of the tumour lysate, the columns were detached and washing and elution was done separately. Eluates from the affinity column were digested using the FASP protocol (Wisniewski et al., 2009, Nat Methods 6:359-62).

Digested samples were separated by on-line reversed-phase nanoscale capillary LC and analyzed by electrospray MS/MS. The experiments were performed on an Eksigent nanoLC system (Eksigent technologies, Dublin, Ireland) coupled to an LTQ Orbitrap Velos mass spectrometer (Thermo Scientific, Bremen, Germany) equipped with a nanoelectrospray ion source (Proxeon Biosystems, Odense, Denmark). Peptides were resuspended in 0.1% FA (formic acid) and loaded from a cooled nanoLC AS-2 autosampler (Eksigent). In order to pre-concentrate and desalt the samples before switching the pre-column in line with the separation column, 10 µl from each sample was loaded onto a reversed-phase ReproSil Pur C18-Aq 5 µm 0.3 × 10 mm trapping cartridge (SGE Analytical Science, Melbourne, Australia), and washed for 5 min at 2.5 µl/min with loading buffer (0.1% FA). The peptides were eluted from a RP ReproSil Pur C18-AQ 3 µm 200 × 0.075 mm (Dr. Maisch GmbH, Ammerbuch-Entringen, Germany) by application of a binary gradient consisting of 2% ACN (acetonitrile) in 0.1% FA (buffer A) and 100% ACN in 0.1% FA (buffer B), with a flow rate of 300 nl/min. Peptides were separated using the following gradient: 0-5 min 2% B, 5-60 min 25% B and 60-88 min 60% B. The column was operated at a constant temperature of 30°C.

The LTQ Orbitrap Velos was operated in positive ionization mode. The MS survey scan was performed in the FT analyzer scanning a window between 250 and 1750 m/z. The resolution was set to 60,000 FWHM at m/z 400 and the automatic gain control (AGC) was set to 500,000 ions. The m/z values triggering MS/MS with a repeat count of 1 were put on an exclusion list for 40 s. The minimum MS signal for triggering MS/MS was set to 800 counts. In all cases, one microscan was recorded. The lock mass option was enabled for both MS and MS/MS mode and the polydimethylcyclosiloxane ions (PDMS, protonated (Si (CH₃)₂O))₆; *m*/*z* 445.120025) were used for internal recalibration of the mass spectra (5, 6). For the HCD, up to the 15 most abundant isotope patterns with charge ≥2 from the survey scan were selected with an isolation window of 2 *m*/*z* fragmented in the C-trap collision cell. Normalized collision energy was set to 40%, the Q value to 0.25 and an activation time to 0.10 ms. Waveform filter was activated. The resulting fragments were detected in the Orbitrap system with a resolution of 7500 FWHM at m/z 400. The maximum ion injection times for the survey scan and the MS/MS scans were 500 ms and 250 ms respectively and the ion target values were set to 1E6 and 4E5, respectively for each scan mode.

The raw files were processed using the Proteome Discoverer 1.3.0.339 software suite (Thermo Scientific). The fragmentation spectra were searched against the SwissProt database (release date: October 19, 2011; 530264 entries) using MASCOT (Perkins DN et al. Probability-based protein identification by searching sequence databases using mass spectrometry data. Electrophoresis. 1999;20:3551-67) as the search engine (v 2.2) with the precursor and fragment mass tolerances set to 10 ppm and 0.1 Da, respectively, and with up to two missed cleavages. Carbamidomethylation of cysteine was set as a fixed modification and oxidation of methionine were chosen as variable modifications for database searching. Both peptide and protein identification were filtered at 1% false discovery rate (FDR) and thus not dependent on the peptide score. Peptides with high and medium confidence were considered. Gene Ontology analyses of the identified proteins were performed. The results obtained for BGal and 011H12 samples were then exported into Excel for manual data interpretation.

### Statistics

In order to choose the most appropriate test, the assumptions of normality and homoscedasticity were evaluated. The small sample size (homing experiments) or the heterogeneous distribution (phage staining) voided these assumptions, therefore the statistical analysis of the data was performed using non-parametric Mann-Whitney U tests. The statistical analysis and the graphic representation using box plots were performed using the SPSS-19 package (IBM) and the XLStat plug-in (XLStat) for MS Excel (Microsoft).

### ELISA

The ability of monovalent and multivalent antibodies to bind human recombinant PSME1, PSME2, HPRT, NDKB (all from Abcam, Cambridge, UK), or CEA (Sigma-Aldrich, St Louis, MO, USA) was studied by ELISA. Maxisorp (NUNC Brand Products, Roskilde, Denmark) plates were coated with specific antigens (1 µg/well) overnight, and after washing and blocking with 200 µl 5% non-fat dry milk in PBS, 100 µl of soluble purified monovalent 011H12 or CEA1 antibody (20 µg/ml), or supernatant from single transfected (pCR3.1-011H12-NC1^{ES}- or pCR3.1-CEA1-NC1^{ES}-) 293T cells were added for 1 h at room temperature, and then 100 µl of anti-myc mAb was added for another h at room temperature. After two washes, 100 µl of biotinylated goat anti-mouse antibody (GE Healthcare) was added for 1 h at room temperature, and then 100 µl of HRP (horseradish peroxidase)-conjugated streptavidin-polymer (Sigma-Aldrich) was added for 30 min at room temperature, after which the plate was washed and developed.

### Surface Plasmon Resonance (SPR) analysis

Experiments were performed at 25°C, using a four-channel BIAcore 3000 optical biosensor system (GE Healthcare). Purified PMSE1 and HRPT, dissolved at 50-100 µg/ml in NaAc 10 mM pH 5.0, were covalently immobilized at 4,000 to 12,000 RU on research grade CM5-sensor chips using the Amine Coupling Kit (GE Healthcare). Unreacted moieties on the surface were blocked with ethanolamine. An activated and blocked flow cell was used as reference surface. HBS-EP buffer (10 mM HEPES pH 7.4, 150 mM NaCl, 3.4 mM EDTA, 0.005% Surfactant P20) was used as sample dilution and running buffer in all experiments. Purified 011H12 and CEA1 antibodies were dialyzed against this buffer before the analysis. Serial dilutions of each antibody, prepared in filtered and degassed buffer, were passed over the immobilized protein surfaces at a flow rate of 10 µl/min. After the association phase, bound analytes were allowed to spontaneously dissociate for surface regeneration. Both, the activated and inactivated surface and the HRPT surface were used as control for the subtraction of bulk effects associated with buffer and analyte concentration changes.

### Immunohistochemistry

Immunohistochemical (IHC) expression of PSME1 was assessed using two prostate tissue microarrays (TMAs), kindly provided by the Tissue Bank Unit of the Spanish National Cancer Research Centre (CNIO), comprising in total 91 different samples, 82 tumoral and 9 normal tissues. IHC was performed on paraffin sections from the TMA blocks using PSME1-specific rabbit polyclonal antibody (Abcam) at 1:400 dilution after antigen retrieval with low pH buffer in Autostainer platform (Dako, Glostrup, Denmark). The immunohistochemical results were evaluated by two independent pathologists. Slides were digitalized using the Mirax Scan (Carl Zeiss AG, Oberkochen, Germany) and pictures were taken with the Pannoramic Viewer software (3D Histech Ltd., Ramsey, NJ, USA). This study has the approval of the Bioethics Committee of the Hospital Universitario Puerta de Hierro and fulfills all ethical requirements.

### Infrared Immunophotodetection in mice bearing PPC-1 xenografts

Imaging was performed as described (Cuesta et al., 2009 cited *supra).* Briefly, when tumours reached a volume of 0.35-0.4 cm³, mice were injected in the tail vein with 50 µl Cy7-labeled antibody solution in PBS. Mice were imaged using the high resolution charge-coupled-device (CCD) cooled digital camera ORCA-2BT (Hamamatsu Photonics France, Massy, France), and Hokawo software (Hamamatsu Photonics), under anesthesia.

### 2. RESULTS

### 2.1 Selection of DAbs

### Repertoire enrichment strategy

In preparation for *in vivo* screening, inventors hypothesized that the ideal repertoire should be moderately enriched against the target organ, but not composed of predominant sequences against a few antigens, thus diverse enough to ensure enrichment against other antigens could be possible. Enrichment of the phage antibody library in phage that binds to cultured tumour cells (*in vitro* strategy) or cell suspensions from freshly excised tumours (*ex vivo* strategy) was compared. To avoid overselection for antibody libraries to vascularly inaccessible tumour antigens not only the increase in the recovery (Fig. 1a) was monitored but also the diversity loss during the rounds of enrichment (Fig. 1b). Sequencing 96 clones from the input and the output of each round of selection confirmed that whereas following *in vivo* selection the repertoire was biased towards a few dominant sequenes, following *ex vivo* selection the phage repertoire was enriched, but still highly diverse.

### Optimizing circulation time and selection of candidates

A circulation time of 15 min is considered to be sufficient to address most of the targets exposed in the endothelium in nonmalignant tissues, but may be too short for dysfunctional tumour vessels. A circulation time of 1 h has been proposed for a filamentous phage peptide library based on the half-life of the phage, although there is evidence that the displayed exogenous protein or peptide can make the circulation half-life as short as 1.5 min and as long as 4.5 h.

A phage pool that had been previously enriched *ex vivo* was administered intravenously and compared the recovery of phage from different organs at different time points after injection [ranging from 15 min to 24 h, using the half-life of wild type phage as a reference at 4 h (27)]. The number of phage recovered from the tumour at 15 min after the injection was approximately 40 times higher than at 24 h; in the non-malignant organs this difference was approximately 100 fold (Fig. 1c, upper panel). This difference is more easily visualized using a value normalized to control phage (Fig. 1c, lower panel). These results suggest that specific accumulation in the target organ is nearly 4 times greater at 24 h than at 15 min.

Having established a 24 h circulation time as optimal for the *in vivo* selections, a sample of clones was sequenced from the input pool, from phage recovered from tumour tissue and from reference organs. Candidate tumour-homing phage clones were appraised based on several criteria: abundance in the target output; similarity between self and consensus CDR's sequences obtained from hierarchical clustering; composition of the (if present) amber stop codons; composition (if present) of common known homing motifs [such as integrin-binding RGD motif, neuropilin-1 binding tissue penetrating C-end Rule motif, CD13 binding NGR motif, etc.] (Table 1).

A computer-based approach was also explored by analyzing the frequency of all possible combinations of three amino acids among the CDR's (given that the framework remains constant and that the interaction between the antibody and the ligand may involve non-contiguous residues) for the *in vivo* output of a single pre-enriched repertoire. Despite the fact that the number of sequences analyzed in this way was limited, we found (Fig. 9) significant differences in the sequences obtained from the tumour (TC) and from the organs (OC) that suggest that deep sequencing would indeed be helpful to either identify new candidates and consensus sequences or to validate the previously selected candidates. The similarity heatmap for the selected sequences and these computed group of consensus sequences (Fig. 10, lower pannel) shows that TC have more triplets of amino acids also present in other TC than in OC. OC have a similar number of triplets also present in TC and OC, and the selected candidates mostly resemble the distribution pattern of TC.

The selected candidate clones were finally evaluated in an *in vivo* play-off (competitive homing) experiment, and compared to a reference irrelevant clone that harbors the anti-β-galactosidase (BGal) specificity. Comparison of the recovery to the input (Fig. 1d, upper panel) or to the average from the normal organs (kidney, liver and heart, Fig. 1d, lower panel) yielded 4 promising phage clones for further analysis.

### Homing assays

The ability of four of the selected clones to home to the tumour was compared to control BGal phage. A fresh preparation of each phage clone was intravenously injected into a group of mice (n = 5 per group), with the circulation time set at 24 h (Fig. 2). In order to validate the reproducibility of the normalized value used, two additional mice were injected a 1:100 dilution of one of the selected clones to compare the total amount of phage recovered and the normalized value for a given specificity and different dosage (Fig. 9). The reference BGal phage (Fig. 2a) showed a baseline accumulation in the tumour, similar to the accumulation in the kidney, and a lower accumulation in both heart and liver. Three of the four selected clones, showed a higher uptake in the tumour (Fig. 2c-e), whereas the remaining clone -that has one of the lowest scores in the heatmap (Fig. 9, d)- accumulated in all the organs (Fig. 2b). Inventors focused on the clone designated as 011H12 for further characterization based on the distribution observed.

### Tissue distribution

Phage may be prone to unspecific aggregation and/or interaction with tissue components accessible from the bloodstream. Therefore it was decided to investigate whether the retention observed for the different phage was specific to the antibody harbored, or merely the result of entrapment of the aggregates on the tumour vascular tree. If that were so, a) a co-localization of endothelial markers with the i.v. administered phage should be expected; b) the differences in the amount of phage recovered should be related to differences in the number of vessels.

The endothelial staining of the tumours from mice dosed with the reference phage, with tumours of mice that have received the 011H12 clone, was compared. The accumulation of the 011H12 phage in the tumour was greater than that of the reference phage when either the total area positive for the phage staining (Fig. 3b), or the relative phage over endothelium area (V, Fig. 3c), were compared (p < 0.0001, Fig. 3d). As expected, there were no differences in the total tumour section area that stained positive for endothelial markers (Fig. 3a). The high dispersion of the values reflects the heterogeneous accumulation of the phage (Fig. 3e): whereas the reference phage is confined to the lumen of the vessels (Fig. 3f, upper row), the 011H12 phage are found also spreading from the vessels into wide areas of surrounding perivascular stroma (Fig. 3f, mid and lower rows). This specific accumulation pattern is not observed in the reference organs where the distribution of the phages is similarly circumscribed to the vessels (Fig. 11). Thus, it suggested that the progressive accumulation of the 011H12 phage in tumour stroma was due to the interaction with a tumour-associated antigen and combination of leaky tumour vessels and long phage circulation time.

### 2.2 Identification of the target of the 011h12 antibody

To identify the target of the 011H12 antibody tumour extracts were subjected to affinity chromatography. Thus, it was coupled in tandem a column with the BGal antibody (Fig. 12) and another column with the 011H12 antibody and bound proteins were identified by nanoLC-MS. Proteins from BGal and 011H12 columns were compared and common proteins (2 or more peptides in the control) were discarded. The remaining proteins were ranked according to the number of peptides found in the eluate from the 011H12 column (Table 1). In order to prioritize the potential candidates, the number of spectra associated with each protein (PSM number) was also considered. Notably, two of the selected proteins were subunits of the 11S proteasome activator complex (PA28): subunit α (PSME) and subunit β (PSME2). To confirm the antigen recognized by the 011H12 antibody, the interaction with the selected candidates was evaluated by ELISA. Soluble purified 011H12 antibody showed reactivity with PSME1 and to a lesser extent with PSME2 (Fig. 4a). No reactivity was observed with the other lower score candidates studied (HPRT and NDKB). The binding of 011H12 antibody to PSME1 was further confirmed by surface plasmon resonance (Fig. 4c). Very rapid on- and off-rates were found, that were too fast to be reliably measured. The weak binding suggests a small interaction surface and implies a minor contribution to the binding energy, compatible with the recognition of a complex epitope that could be partially exposed in the purified PSME1 and whose integrity depends on the presence of other PA28 subunits. In fact, it was found that the ELISA signal slightly increased when 011H12 antibody was added in wells coated with both PSME1 and PSEM2 (Fig. 4b).

**Table 2**

| **Potential targets of 011H12 identified by MS** | | | | |
|---|---|---|---|---|
| **Accession** | **Description** | **Coverage, %** | **Peptide number** | **PSM¹ number** |
| Q06323 | Proteasome activator complex subunit 1 | 53.8 | 13 | 64 |
| P00492 | Hypoxanthine-guanine phosphoribosyltransferase | 50.9 | 9 | 34 |
| Q9UL46 | Proteasome activator complex subunit 2 | 28.9 | 7 | 25 |
| P22392 | Nucleoside diphosphate kinase B | 44.7 | 7 | 25 |
| Q15436 | Protein transport protein Sec23A | 9.4 | 6 | 18 |
| P15531 | Nucleoside diphosphate kinase A | 41.5 | 6 | 20 |
| Q9ULA0 | Aspartyl aminopeptidase | 15.4 | 6 | 25 |
| P02768 | Serum albumin | 8.7 | 6 | 13 |
| Q96CN7 | Isochorismatase domain-containing protein 1 | 17.8 | 4 | 15 |
| P06730 | Eukaryotic translation initiation factor 4E | 22.1 | 4 | 16 |

| | | | | |
|---|---|---|---|---|
| ¹PSM (peptide spectral match) number: number of spectra associated with a protein. | | | | |

### 3. Localization of PSME1 expression in PPC-1 tumour xenografts

To investigate the localization of PSME1 in tumours, sections of PPC-1 tumour xenografts were stained for PSME2 and CD31. PSME1 is heterogeneously distributed: most of the blood vessels show a positive staining while there are wide areas negative for CD31 that are PSME1 positive (Fig. 13). Distribution of PSME1 in the tumours that had received the control or the 011H12 phage (Fig. 5) was then studied. It was found that the distribution of PSME1 is similar in either group of animals, and that there are areas of co-localization between PSME1 and the i.v. administered 011H12 phage. This distribution is compatible with a possible target for the 011H12 antibody.

To study the accessibility of PSME1 expressed in prostate tumours the anti-PSME1 antibody and a rabbit control IgG antibody were labeled with Cy7 and intravenously injected into PPC-1 tumour-bearing mice (n = 3 per group). Both antibodies showed a rapid renal clearance, with peak signal intensity at 3 h and no detectable bladder signal at 24 h post-injection (Fig. 6a). The anti-PSME1 antibody localized in the tumours, whereas the control rabbit IgG showed no detectable localization (Fig. 6b). Maximum resolution was achieved at about 24 h. *Ex vivo* imaging of the organs further confirms the specific accumulation of the anti-PSME1 antibody in the tumour and liver, while showing a similar uptake by the kidney for both control and anti-PSME1 antibodies (Fig. 6c-d).

### 4. Validation of PSME1 as biomarker in human primary prostatic carcinomas

Validation of a candidate protein biomarker in human normal and tumoural tissue samples is an essential step in moving from the initial discovery to possible applications. Therefore, PSME1 expression in human prostate sections was studied by immunohistochemical staining on two distinct prostate tumour specific tissue microarrays (TMA) containing a total of 91 human tissues, 82 of them were tumoural samples and 9 with normal tissue. Representative immunostaining patterns of PSME1 in normal and tumoural tissues are shown in Fig. 7. In normal prostatic glands the anti-PSME1 antibody stained strongly only basal cells, with weak cytoplasmic positivity in luminal epithelial cells. Most blood vessels were positive with occasional staining in surrounding stromal cells. In contrast, prostate cancer tissues showed much higher PSME1 expression levels than the corresponding normal prostate tissue. Remarkably, a strong staining was observed in tumoural luminal cells, and in the stromal compartment, where extracellular PSME1-positive deposits were evident. Basal cells were not observed as the loss of these cells is a common characteristic of prostate carcinogenesis. Blood vessels remained positive for PSME1 staining.

## Claims

1. An *in vitro* method for diagnosing prostate cancer in a subject comprising
a) detecting the presence of at least one subunit of PA28 in a prostate tissue sample from said subject,
wherein the detection of said at least one subunit of PA28 on the cell surface is indicative of said subject having prostate cancer, or
b) quantifying the expression level of at least one subunit of PA28 in a prostate tissue sample from said subject and comparing the level of said at least one subunit of PA28 in the sample with a reference value,
wherein a level of said at least one subunit of PA28 in said sample higher than a reference value is indicative of said subject having prostate cancer, or
c) quantifying the expression level of at least one subunit of PA28 in a biological fluid sample from said subject, and comparing said level of at least one subunit of PA28 with a reference value
wherein a level of said at least one subunit of PA28 in said sample higher than a reference value is indicative of said subject having prostate cancer.

2. Method according to claim 1, comprising
a) detecting the presence of one subunit of PA28 in a prostate tissue sample from said subject,
wherein the detection of said one subunit of PA28 on the cell surface is indicative of said subject having prostate cancer, or
b) quantifying the expression level of one subunit of PA28 in a prostate tissue sample from said subject and comparing the level of said one subunit of PA28 in the sample with a reference value,
wherein a level of said one subunit of PA28 in said sample higher than a reference value is indicative of said subject having prostate cancer, or
c) quantifying the expression level of one subunit of PA28 in a biological fluid sample from said subject, and comparing said level of one subunit of PA28 with a reference value
wherein a level of said one subunit of PA28 in said sample higher than a reference value is indicative of said subject having prostate cancer.

3. Method according to any of claims 1 or 2, wherein the detection in (a) or is performed by means of a technique selected from the group consisting of immunohistochemistry and immunofluorescence.

4. Method according to any of claims 1 or 2, wherein the quantification in (b) and (c) is performed by means of a technique selected from the group consisting of immunohistochemistry, immunofluorescence, real time PCR (RT-PCR), and *in situ* hybridization.

5. Method according to any of claims 1 to 4, wherein the prostate tissue sample is a biopsy.

6. Method according to any of claims 1 to 4, wherein the biological fluid sample is a blood, serum or plasma sample.

7. A therapeutic agent that binds specifically to at least one subunit of PA28 for use in the treatment of a subject suffering from prostate cancer, wherein said therapeutic agent comprises a first component selected from the group consisting of
- an antibody that binds specifically to at least one subunit of PA28;
- an antibody fragment that binds specifically to at least one subunit of PA28; and
- an antibody-like molecule that binds specifically to at least one subunit of PA28;

8. Therapeutic agent according to claim 7, further comprising a second component selected from the group consisting of a radionuclide, a toxin, an enzyme, a cytokine, an interleukin, and an angiogenesis inhibitor.

9. Method according to any of claims 1 to 6, or therapeutic agent according to any of claims 7 or 8, wherein the PA28 subunit is selected from the group consisting of PSME1 and PSME2, and is preferably PSME1.

10. A kit for diagnosing prostate cancer comprising at least a reagent for performing the method according to any of claims 1 to 6, 9 or 10, wherein said reagent comprises an antibody which specifically binds to at least one subunit of PA28, and/or a reagent specific for the mRNA encoding at least one subunit of PA28.

11. A kit comprising a therapeutic agent according to any of claims 7 to 10.

12. PA28 or a subunit thereof as a prostate cancer marker.

13. A domain antibody which specifically binds to at least one subunit of PA28 and comprises the variable domain of an antibody heavy chain, wherein said domain antibody is selected from the group of domain antibodies fragments listed in Table 1 or functionally active variants or fragments thereof.

14. A domain antibody according to claim 13, comprising a heavy chain CDR1 (HCDR1) with amino acid sequence SEQ ID NO: 13, a HCDR2 with amino acid sequence SEQ ID NO: 14, and a HCDR3 with amino acid sequence SEQ ID NO: 15.

15. An antibody, or a fragment thereof, which specifically binds to at least one subunit of PA28, comprising the domain antibody according to any of claims 13 or 14.
